# EUROPEAN PATENT APPLICATION

(11) **EP 1 795 599 A1**
(43) Date of publication of application: **13.06.2007**
(21) Application number: 05111928.7
(22) Date of filing: 09.12.2005
(51) Int. Cl.: C12N 15/82, C12N 15/87, C07K 14/705, C12N 5/06

(54) **Methods for generating antigen-specific effector T cells**

(71) Applicant: Schuler, Gerold, Prof. Dr., 91080 Spardorf (AT)
(72) Inventor: Schuler, Gerold, Prof.Dr., 91080 Spardorf (DE); Dörrie, Jan, Dr., 90409 Nürnberg (DE); Schaft, Niels, Dr., 91074 Herzogenaurach (DE)
(74) Representative: von Kreisler Selting Werner

(57) **Abstract**

The invention relates to T cells transiently transfected with RNA, especially RNA encoding a T cell receptor and/or FoxP3, and to methods of transfecting T cells with RNA by electroporation. Compositions of the invention include an effector T cell transiently transfected with RNA encoding a T cell receptor (TCR) specific for an antigen, wherein the T cell demonstrates effector function specific for cells presenting the antigen in complex with an MHC molecule. T_{reg} cells comprising an exogenous RNA encoding FoxP3 are also provided. The transfected T cells are useful for immunotherapy, particularly in the treatment of tumors, pathogen infection, autoimmune disease, transplant rejection and graft versus host disease.

SEQUENCE LISTING

<110> SCHULER, Gerold

<120> Method for Generating Antigen-Specific Effector T Cells

<130> 052954ep

<140> EP 05111928.7
<141> 2005-12-09

<160> 10

<170> PatentIn version 3.3

<210> 1
<211> 844
<212> DNA
<213> Homo sapiens

<400> 1
atggcatcca ttcgagctgt atttatattc ctgtggctgc agctggactt ggtgaatgga 60

gagaatgtgg agcagcatcc ttcaaccctg agtgtccagg agggagacag cgctgttatc 120

aagtgtactt attcagacag tgcctcaaac tacttccctt ggtataagca agaacttgga 180

aaaggacctc agcttattat agacattcgt tcaaatgtgg gcgaaaagaa agaccaacga 240

attgctgtta cattgaacaa gacagccaaa catttctccc tgcacatcac agagacccaa 300

cctgaagact cggctgtcta cttctgtgca gcaagtactt cgggtggtac tagctatgga 360

aagctgacat ttggacaagg gaccatcttg actgtccatc caaatatcca gaaccctgac 420

cctgccgtgt accagctgag agactctaaa tccagtgaca agtctgtctg cctattcacc 480

gattttgatt ctcaaacaaa tgtgtcacaa agtaaggatt ctgatgtgta tatcacagac 540

aaaactgtgc tagacatgag gtctatggac ttcaagagca acagtgctgt ggcctggagc 600

aacaaatctg actttgcatg tgcaaacgcc ttcaacaaca gcattattcc agaagacacc 660

ttcttcccca gcccagaaag ttcctgtgat gtcaagctgg tcgagaaaag ctttgaaaca 720

gatacgaacc taaactttca aaacctgtca gtgattgggt tccgaatcct cctcctgaaa 780

gtggccgggt ttaatctgct catgacgctg cggctgtggt ccagctgagg atccctcgag 840

agag 844

<210> 2
<211> 280
<212> PRT
<213> Homo sapiens

<400> 2

Met Ala Ser Ile Arg Ala Val Phe Ile Phe Leu Trp Leu Gln Leu Asp
1               5                   10                  15

Leu Val Asn Gly Glu Asn Val Glu Gln His Pro Ser Thr Leu Ser Val
            20                  25                  30

Gln Glu Gly Asp Ser Ala Val Ile Lys Cys Thr Tyr Ser Asp Ser Ala

35                  40                  45

Ser Asn Tyr Phe Pro Trp Tyr Lys Gln Glu Leu Gly Lys Gly Pro Gln
    50                  55                  60

Leu Ile Ile Asp Ile Arg Ser Asn Val Gly Glu Lys Lys Asp Gln Arg
65                  70                  75                  80

Ile Ala Val Thr Leu Asn Lys Thr Ala Lys His Phe Ser Leu His Ile
                85                  90                  95

Thr Glu Thr Gln Pro Glu Asp Ser Ala Val Tyr Phe Cys Ala Ala Ser
            100                 105                 110

Thr Ser Gly Gly Thr Ser Tyr Gly Lys Leu Thr Phe Gly Gln Gly Thr
        115                 120                 125

Ile Leu Thr Val His Pro Asn Ile Gln Asn Pro Asp Pro Ala Val Tyr
    130                 135                 140

Gln Leu Arg Asp Ser Lys Ser Ser Asp Lys Ser Val Cys Leu Phe Thr
145                 150                 155                 160

Asp Phe Asp Ser Gln Thr Asn Val Ser Gln Ser Lys Asp Ser Asp Val
                165                 170                 175

Tyr Ile Thr Asp Lys Thr Val Leu Asp Met Arg Ser Met Asp Phe Lys
            180                 185                 190

Ser Asn Ser Ala Val Ala Trp Ser Asn Lys Ser Asp Phe Ala Cys Ala
        195                 200                 205

Asn Ala Phe Asn Asn Ser Ile Ile Pro Glu Asp Thr Phe Phe Pro Ser
    210                 215                 220

Pro Glu Ser Ser Cys Asp Val Lys Leu Val Glu Lys Ser Phe Glu Thr
225                 230                 235                 240

Asp Thr Asn Leu Asn Phe Gln Asn Leu Ser Val Ile Gly Phe Arg Ile
                245                 250                 255

Leu Leu Leu Lys Val Ala Gly Phe Asn Leu Leu Met Thr Leu Arg Leu
            260                 265                 270

Trp Ser Ser Gly Ser Leu Glu Arg
        275                 280

<210>  3
<211>  949
<212>  DNA
<213>  Homo sapiens

<400> 3
atgggccccc agctccttgg ctatgtggtc ctttgccttc taggagcagg ccccctggaa 60

gcccaagtga cccagaaccc aagatacctc atcacagtga ctggaaagaa gttaacagtg 120

acttgttctc agaatatgaa ccatgagtat atgtcctggt atcgacaaga cccagggctg 180

ggcttaaggc agatctacta ttcaatgaat gttgaggtga ctgataaggg agatgttcct 240

gaagggtaca aagtctctcg aaaagagaag aggaatttcc ccctgatcct ggagtcgccc 300

agccccaacc agacctctct gtacttctgt gccagcagtt tgggggagctc ctacgagcag 360

tacttcgggc cgggcaccag gctcacggtc acagaggacc tgaaaaacgt gttcccaccc 420

gaggtcgctg tgtttgagcc atcagaagca gagatctccc acacccaaaa ggccacactg 480

gtgtgcctgg ccacaggctt ctaccccgac cacgtggagc tgagctggtg ggtgaatggg 540

aaggaggtgc acagtggggt cagcacagac ccacagcccc tcaaggagca gcccgccctc 600

aatgactcca gatactgcct gagcagccgc ctgagggtct cggccacctt ctggcagaac 660

ccccgcaacc acttccgctg tcaagtccag ttctacgggc tctcggagaa tgacgagtgg 720

acccaggata gggccaaacc tgtcacccag atcgtcagcg ccgaggcctg gggtagagca 780

gactgtggct tcacctccga gtcttaccag caaggggtcc tgtctgccac catcctctat 840

gagatcttgc tagggaaggc caccttgtat gccgtgctgg tcagtgccct cgtgctgatg 900

gccatggtca agagaaagga ttccagaggc tagcgatccc tcgagagag 949

<210> 4
<211> 315
<212> PRT
<213> Homo sapiens

<400> 4

Met Gly Pro Gln Leu Leu Gly Tyr Val Val Leu Cys Leu Leu Gly Ala
1               5                   10                  15

Gly Pro Leu Glu Ala Gln Val Thr Gln Asn Pro Arg Tyr Leu Ile Thr
            20                  25                  30

Val Thr Gly Lys Lys Leu Thr Val Thr Cys Ser Gln Asn Met Asn His
        35                  40                  45

Glu Tyr Met Ser Trp Tyr Arg Gln Asp Pro Gly Leu Gly Leu Arg Gln
    50                  55                  60

Ile Tyr Tyr Ser Met Asn Val Glu Val Thr Asp Lys Gly Asp Val Pro
65                  70                  75                  80

Glu Gly Tyr Lys Val Ser Arg Lys Glu Lys Arg Asn Phe Pro Leu Ile
                85                  90                  95

Leu Glu Ser Pro Ser Pro Asn Gln Thr Ser Leu Tyr Phe Cys Ala Ser
            100                 105                 110

Ser Leu Gly Ser Ser Tyr Glu Gln Tyr Phe Gly Pro Gly Thr Arg Leu
        115                 120                 125

Thr Val Thr Glu Asp Leu Lys Asn Val Phe Pro Pro Glu Val Ala Val
    130                 135                 140

Phe Glu Pro Ser Glu Ala Glu Ile Ser His Thr Gln Lys Ala Thr Leu
145                 150                 155                 160

Val Cys Leu Ala Thr Gly Phe Tyr Pro Asp His Val Glu Leu Ser Trp
                165                 170                 175

Trp Val Asn Gly Lys Glu Val His Ser Gly Val Ser Thr Asp Pro Gln
            180                 185                 190

Pro Leu Lys Glu Gln Pro Ala Leu Asn Asp Ser Arg Tyr Cys Leu Ser
        195                 200                 205

Ser Arg Leu Arg Val Ser Ala Thr Phe Trp Gln Asn Pro Arg Asn His
    210                 215                 220

Phe Arg Cys Gln Val Gln Phe Tyr Gly Leu Ser Glu Asn Asp Glu Trp
225                 230                 235                 240

Thr Gln Asp Arg Ala Lys Pro Val Thr Gln Ile Val Ser Ala Glu Ala
                245                 250                 255

Trp Gly Arg Ala Asp Cys Gly Phe Thr Ser Glu Ser Tyr Gln Gln Gly
            260                 265                 270

Val Leu Ser Ala Thr Ile Leu Tyr Glu Ile Leu Leu Gly Lys Ala Thr
        275                 280                 285

Leu Tyr Ala Val Leu Val Ser Ala Leu Val Leu Met Ala Met Val Lys
    290                 295                 300

Arg Lys Asp Ser Arg Gly Arg Ser Leu Glu Arg
305                 310                 315

<210> 5
<211> 9
<212> PRT
<213> Homo sapiens

<400> 5

Glu Ala Asp Pro Thr Gly His Ser Tyr
1               5

<210> 6
<211> 9
<212> PRT
<213> Homo sapiens

<400>  6

Leu Ala Gly Ile Gly Ile Leu Thr Val
1               5

<210>  7
<211>  9
<212>  PRT
<213>  Homo sapiens

<400>  7

Ala Ala Gly Ile Gly Ile Leu Thr Val
1               5

<210>  8
<211>  10
<212>  PRT
<213>  Homo sapiens

<400>  8

Phe Leu Thr Pro Lys Lys Leu Gln Cys Val
1               5                   10

<210>  9
<211>  35
<212>  DNA
<213>  artificial

<220>
<223>  Primer

<400>  9
ctctggatcc atgggccccc agctccttgg ctatg                                    35

<210>  10
<211>  38
<212>  DNA
<213>  artificial

<220>
<223>  Primer

<400>  10
ctctctcgag ggatcgctag cctctggaat cctttctc                                38

## Description

### FIELD OF THE INVENTION

The invention relates to T cells transiently transfected with RNA, especially RNA encoding a T cell receptor and/or FoxP3, and to methods of transfecting T cells with RNA by electroporation. The transfected T cells are useful for immunotherapy, particularly in the treatment of tumors, pathogen infection, autoimmune disease, transplant rejection and graft versus host disease.

### BACKGROUND

Cytotoxic T lymphocytes (CTL) play a major role in the control of tumor growth, and are, therefore, of great importance in cellular strategies for immunotherapy of cancer [19]. Early attempts to adoptively transfer tumor-infiltrating lymphocytes (TIL) were unsatisfactory, because the transferred cells were often non-specific and did not persist for long periods of time, most probably due to the fact that such TIL can have an anergic phenotype or are incapable of homing to tumor sites [10, 11]. Adoptive transfer of *in vitro* expanded autologous tumor-specific CTL has been shown to be effective in eradication of tumors in patients with metastatic melanoma [9, 13, 18, 20, 31]. Unfortunately, not all patients mount a detectable *in vivo* cytotoxic T cell response to their tumors. In fact, isolation and/or expansion of lytic tumor-specific T cells has only been possible in a fraction of patients, most likely due to the fact that, notably in tumor patients, the peripheral T cell repertoire is usually devoid of high-avidity tumor-specific CTL due to thymic selection [28] or other tolerance mechanisms [30]. In addition, these cells and *in vitro* generated tumor-specific T cells only have a limited life-span, and expansion of such T cells to therapeutic doses is often not feasible [2, 3, 30].

Alternatively, since CTL specificity is exclusively dictated by the T cell receptor (TCR), autologous T cells retrovirally transduced with a tumor-specific TCR were used for adoptive transfer. Reprogramming of T cells with a tumor specificity by retroviral transduction has already been shown *in vitro* for several antigens, e.g. MART-1 [7], MAGE-1 [29], MDM2 [27], gp100 [16, 24] and tyrosinase [21]. These autologous T cells were easily expanded to therapeutic doses. However, retroviral transduction poses the threat of irreversible genetic manipulation of autologous cells, as the provirus can integrate at random in the genome of the transduced cells. Thus, it can also integrate into genes involved in cell cycle control, and subsequently disturb cell growth (i.e. insertional mutagenesis). Data of a gene therapy clinical trial in severe combined immunodeficiency (SCID), in which autologous hematopoietic stem cells were retrovirally transduced with a vector containing a gene encoding the common γ chain, which is defective in SCID patients, showed that the provirus integrated in the LMO-2 oncogene, causing leukemia-like symptoms [6, 12, 15]. In addition, retroviral transduction cannot be done with resting, non-dividing T cells [30], but rather requires the stimulation of T cells for several days prior to transduction.

Thus, there is a long-felt need for methods to effectively transfer antigen-specific TCR function to T cells, without the need for retroviral vectors or other forms of transfection which could result in alterations to the host genome. The present invention fulfills this need and provides additional advantages as well.

### SUMMARY OF THE INVENTION

The inventors have discovered improved methods for electroporation of RNA into T cells, especially purified CD8⁺ or CD4⁺ cells. The improved methods make possible the functional transfer of TCR into isolated T cells by RNA electroporation, and subsequent cryoconservation. The methods of the invention avoid the disadvantages of retroviral transduction, and form a new strategy for the immunotherapy of cancer, pathogen infection, autoimmunity, transplantation and graft versus host disease.

In one aspect, the invention provides a composition comprising an effector T cell transiently transfected with RNA encoding a T cell receptor (TCR) specific for an antigen, wherein the T cell demonstrates effector function specific for cells presenting the antigen in complex with an MHC molecule. In preferred embodiments, the effector function is cytotoxicity. The effector T cell compositions of the invention may be used for the production of a medicament for immunotherapy.

In another aspect, the invention provides a method for imparting a new antigen specificity to a T cell, comprising electroporating a composition comprising purified CD8+ or purified CD4+ T cells with RNA encoding a TCR receptor specific for an antigen.

In still another aspect, the invention provides a method for imparting a new antigen specificity to T cells, comprising: electroporating resting T cells with RNA encoding a TCR specific for an antigen.

In another aspect, the invention provides a method for transiently transfecting T cells, comprising electroporating T cells with RNA a field strength of 100V/mm-150V/mm for 2-10 ms using a square wave pulse, wherein the T cells have not been stimulated in vitro by PHA or OKT3 prior to electroporation.

In a further aspect, the invention provides a method of imparting antigen-specific T cell effector function to a subject, comprising administering a T cell transiently transfected with RNA encoding a TCR specific for an antigen, wherein the T cell demonstrates effector function for cell presenting the antigen in complex with an MHC molecule.

In another aspect, the invention provides a T_{reg} cell comprising an exogenous RNA encoding FoxP3. The invention provides methods for making a T_{reg} cell, comprising transfecting a CD4+ T cell with a nucleic acid encoding FoxP3. The FoxP3 transfected T cells can be used for the production of a medicament for immunotherapy, and effective amounts can be administered to patients.

### BRIEF DESCRIPTION OF THE DRAWINGS

For a more complete understanding of the features and advantages of the present invention, reference is now made to the detailed description of the invention along with the accompanying figures and in which:

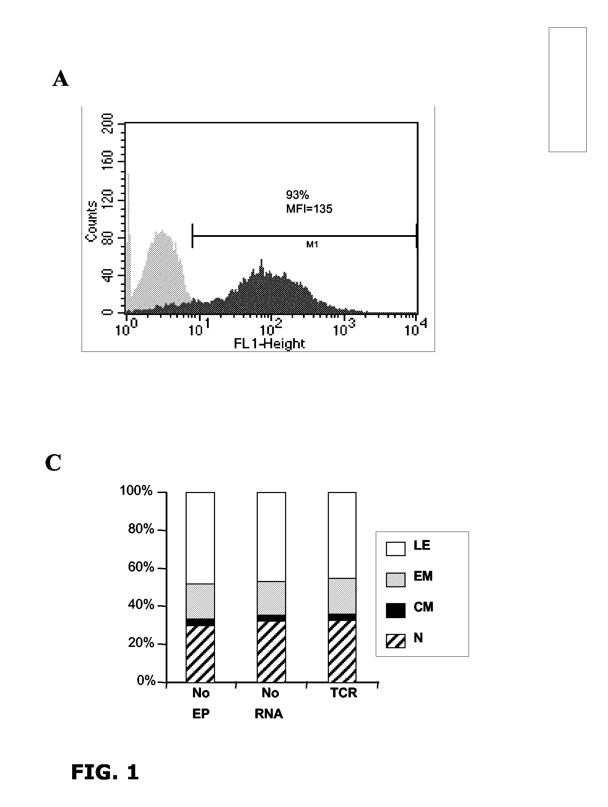

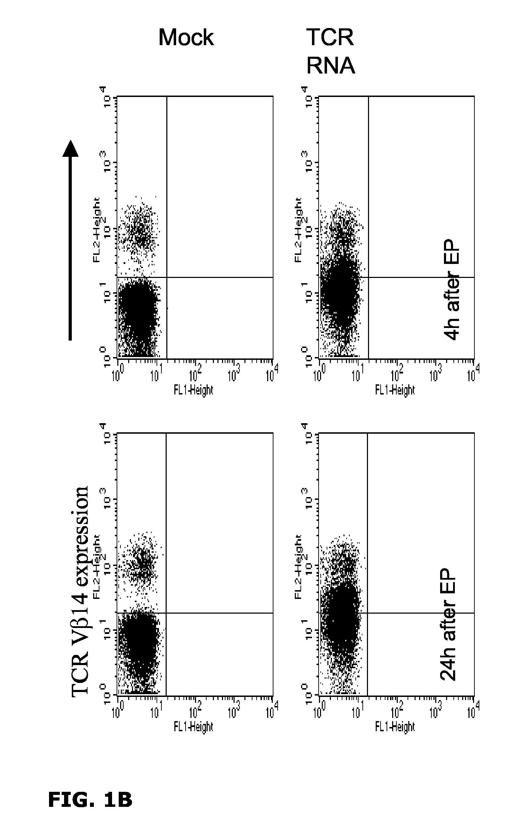

**Figure 1** shows EGFP expression of RNA-transfected CD8⁺ T cells. a) CD8⁺ T cells were electroporated with EGFP RNA, and EGFP expression in these cells was determined by FACS analysis 4h after electroporation (black histogram). CD8⁺ T cells electroporated without RNA served as negative control (gray histogram). b) CD8+ T cells were electroporated with TCR α and β chain RNA (TCR RNA), and TCR Vβ14 surface expression on these cells was determined by FACS analysis 4h and 24h after electroporation (EP). CD8⁺ T cells electroporated without RNA served as negative control (Mock). c) The influence of electroporation and TCR expression on T-cell phenotype was examined by CCR7 and CD45RA staining 24h after electroporation. Assignment of T cell phenotype was as follows: lytic effectors (LE): CD45RA⁺/CCR7⁻, effector memory (EM): CD45RA⁻/CCR7⁻, central memory (CM): CD45RA⁻/CCR7⁺, naive (N): CD45RA⁺/CCR7⁺. All data are representative for three standardized independent experiments.

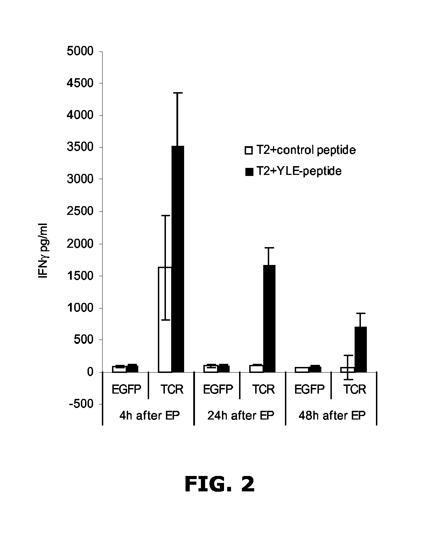

**Figure 2** shows that TCR RNA-transfected T cells specifically produce IFNγ after stimulation with peptide-loaded target cells. CD8⁺ T cells were electroporated with EGFP RNA (EGFP) or RNA coding for the TCR α and β chain (TCR), and were used as effector cells in IFNγ-production assays, 4h, 24h and 48h after electroporation (EP). Irradiated T2 cells either loaded with a control peptide (white bars) or gp100₂₈₀₋₂₈₈ peptide (YLE-peptide, black bars) were used as stimulator cells, and IFNγ production was measured in supernatants in an ELISA, and are expressed in pg/ml. Average values of triplicates ± SD are shown. The effector to stimulator cell ratio was 1:1. Data of one (out of three) representative T cell donor are shown.

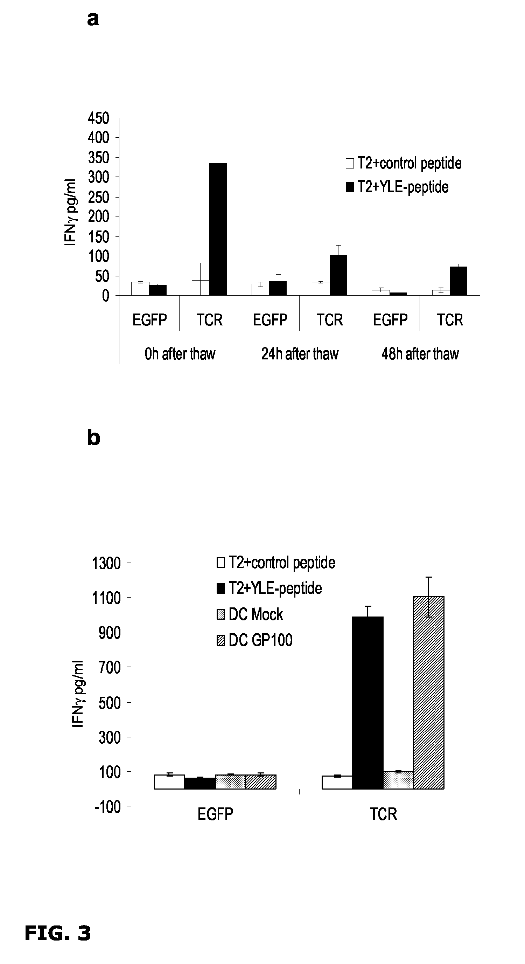

**Figure 3** shows that TCR RNA-transfected T cells can be cryopreserved without loss of IFNγ production capacity. CD8⁺ T cells were electroporated with EGFP RNA (EGFP) or RNA coding for the TCR α and β chain (TCR), and were cryopreserved 4h after electroporation. These T cells were used as effector cells in IFNγ-production assays, 0h (a and b), 24h (a) and 48h (a) after thawing. Irradiated T2 cells either loaded with a control peptide (white bars) or gp100₂₈₀₋₂₈₈ peptide (YLE-peptide, black bars) (a and b), and mock-electroporated DC (Mock, grey bars) or DC electroporated with gp100 RNA (GP100, diagonally-striped bars) (b) were used as stimulator cells, and IFNγ production was measured in supernatants in an ELISA, and are expressed in pg/ml. Average values of triplicates ± SD are shown. The effector to stimulator cell ratio was 1:1. Data of one (out of three) representative T cell donor are shown.

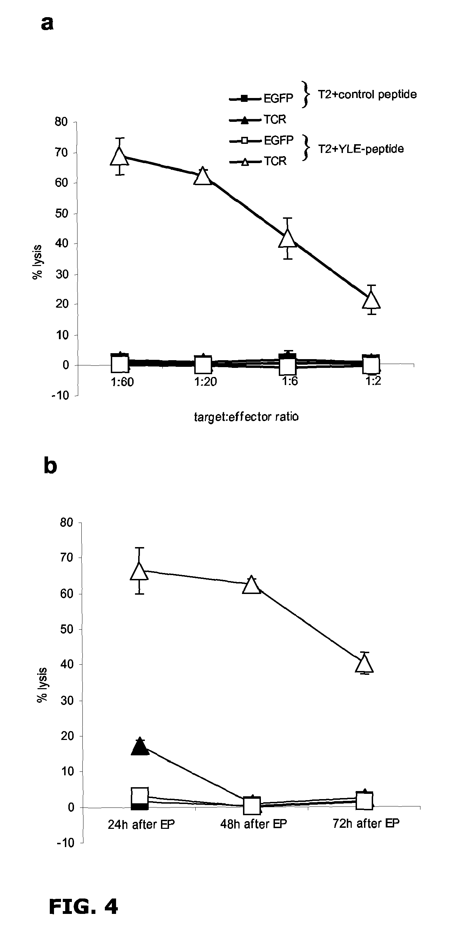

**Figure 4** shows that TCR RNA-transfected T cells specifically lyse peptide-loaded target cells. CD8⁺ T cells were electroporated with EGFP RNA (EGFP, squares) or RNA coding for the TCR α and β chain (TCR, triangles), and were used as effector cells in standard 4h cytotoxicity assays, 24h, 48h and 72h after electroporation (EP). T2 cells either loaded with a control peptide (closed symbols) or gp100₂₈₀₋₂₈₈ peptide (YLE-peptide, open symbols) were used as target cells, and % lysis was calculated (see Materials & Methods section for more details). The target to effector cell ratio was 1:60, 1:20, 1:6 and 1:2 (a, 48h time-point is shown), or 1:20 (b, time-course is shown). Average values of triplicates ± SD are shown. Data of one (out of three) representative T cell donor are shown.

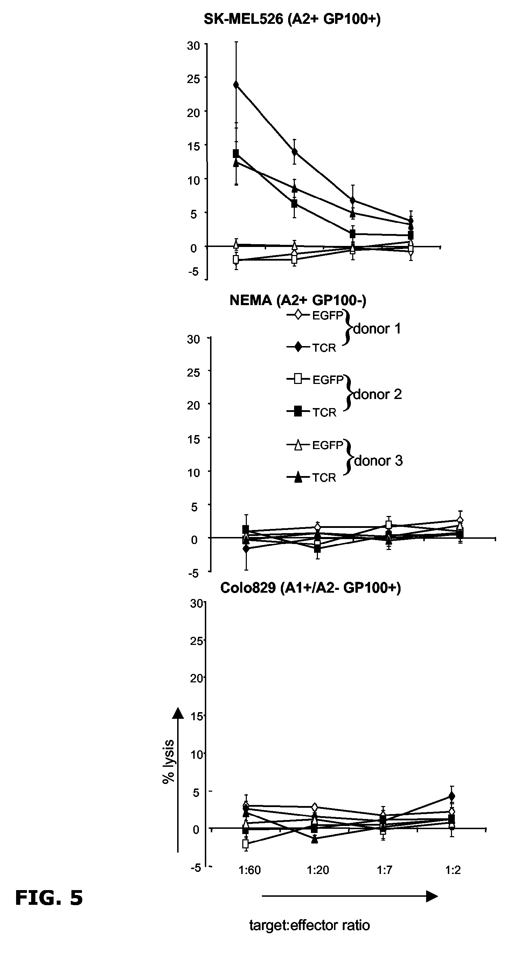

**Figure 5** shows that TCR RNA-transfected T cells specifically lyse a melanoma cell line. CD8⁺ T cells of 3 donors were electroporated with EGFP RNA (EGFP, open symbols) or RNA coding for the TCR α and β chain (TCR, closed symbols), and were used as effector cells in standard 4h cytotoxicity assays 24h after electroporation. The melanoma cell lines SK-MEL526 (HLA-A2⁺/gp100⁺), NEMA (HLA-A2⁺/gp100⁻), and Colo829 (HLA-A1⁺/A2⁻/gp100⁺) were used as target cells, and % lysis was calculated. Average values of triplicates ± SD are shown. The target to effector cell ratio was 1:60, 1:20, 1:6 and 1:2.

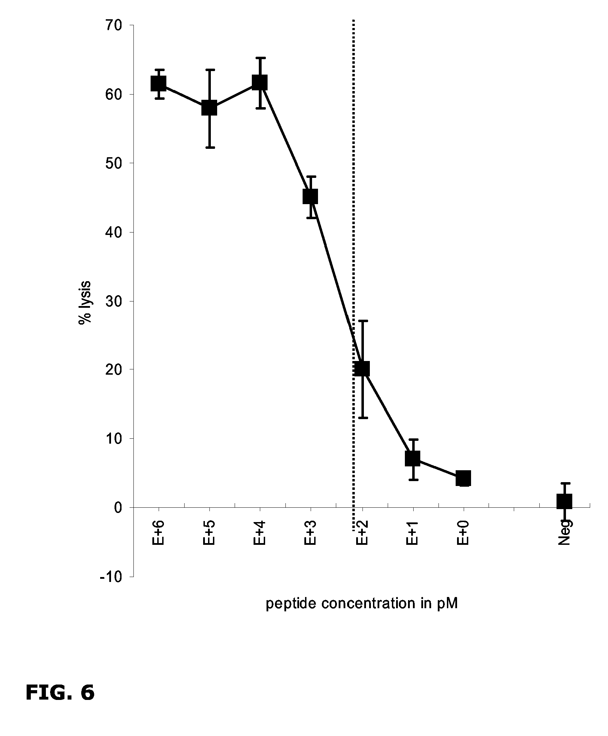

**Figure 6** shows that TCR RNA-transfected T cells have a similar cytolytic capacity as retrovirally transduced T cells, which approximates cytolytic efficiency of the parental CTL clone. CD8⁺ T cells were electroporated with EGFP RNA (Neg) or RNA coding for the TCR α and β chain, and were used as effector cells in cytotoxicity assays 24h after electroporation. T2 cells loaded with different concentrations of gp100₂₈₀₋₂₈₈ peptide (as indicated) were used as target cells, and % lysis was calculated. The target to effector cell ratio was 1:15. The peptide concentration corresponding to 50% of the maximum lysis (ED₅₀), used to measure the cytolytic efficiency, is indicated by the dotted line. Average values of triplicates ± SD are shown. Data of one (out of five) representative T cell donor is shown.

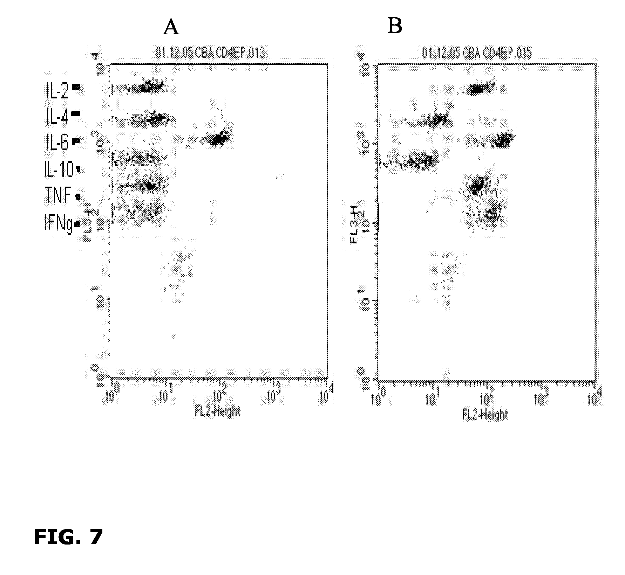

**Figure 7** shows the measurement of 6 different cytokines at once using the BD bead array. Each cloud represents on cytokine. The further to the right (increase of FL2 signal) the higher the cytokine concentration. A: Supernatant from TCR transfected CD4+ cells on control DC. B: Supernatant from TCR transfected CD4+ cells on DC pulsed with the corresponding peptide. Note: the IL-6 is produced by the DC not the T cells. To determine the exact concentrations, a standard curve has to be generated.

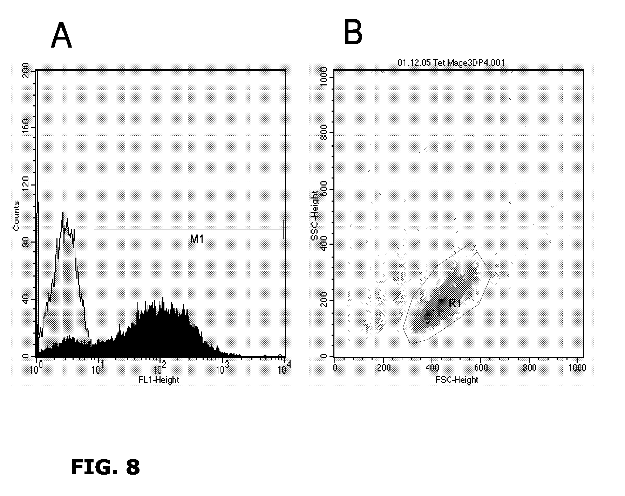

**Figure 8** shows the results of FACS-analysis of RNA-transfected CD4+ cells 24h after electroporation. A: CD4+ cells were transfected with GFP-RNA (black histogram). Here the TCR-RNA transfected cells served as negative control (grey histogram). B: The FSC/SSC of the cells was used to indicate the cells condition, and more than 95% of the cells were found in the "life gate" (R1).

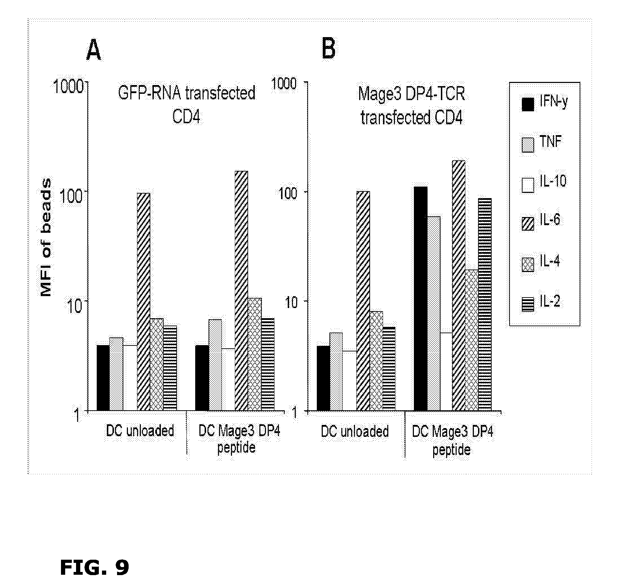

**Figure 9** shows the results of transfection of CD4+ T cells with GFP-RNA (A) or with RNA coding for a Mage3-DP4 specific TCR (B). As negative control target, unloaded autologous DC were used. As specific target, the Mage3-DP4 peptide was loaded on the DC. After 20 h the cytokine concentration in the supernatants was measured by CBA. Only the MFI of the beads was determined, which allows semi-quantitative comparison of the concentrations (but is quite close to proportionality). Note that the IL-6 is produced by the DC, not the T cells.

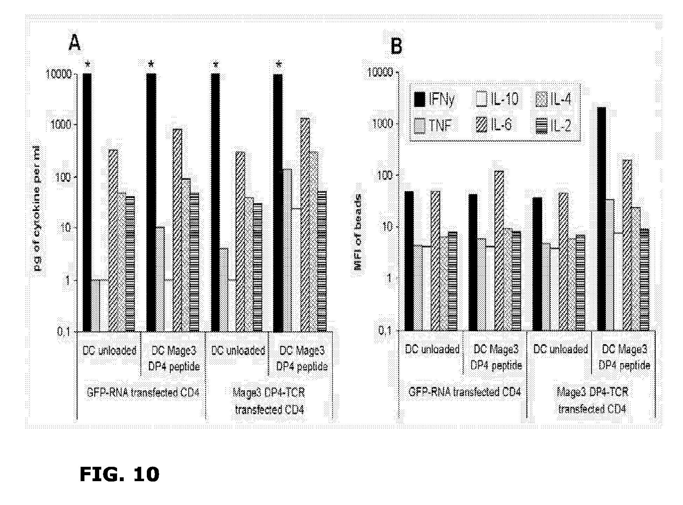

**Figure 10**: CD4+ cells were transfected with GFP-RNA or with RNA coding for a Mage3-DP4 specific TCR. As negative control target, unloaded autologous DC were used. As specific target, the Mage3-DP4 peptide was loaded on the DC. After 44 h the cytokine concentration in the supernatants was measured by CBA (A). Since IFNγ concentrations were out of scale (*), the MFI of the beads is also depicted (B).

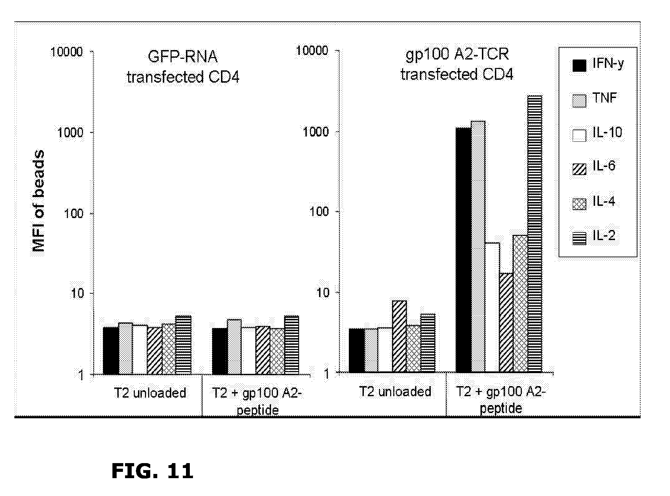

**Figure 11**: CD4+ cells were transfected with GFP-RNA (A) or with RNA coding for the gp100-A2 specific TCR (B). As negative control target, unloaded T2 cells were used. As specific target, the gp100-A2 peptide was loaded on the T2 cells. After 20 h the cytokine concentration in the supernatants was measured by CBA. Only the MFI of the beads was determined, which allows semi-quantitative comparison of the concentrations (but is quite close to proportionality).

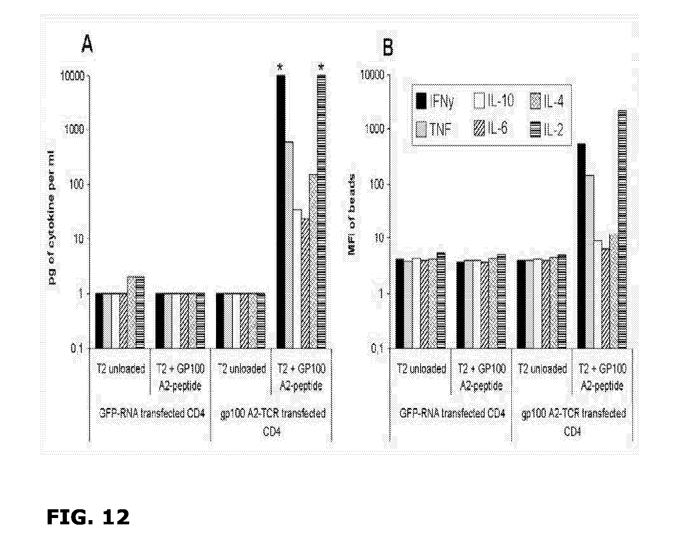

**Figure 12**: CD4+ cells were transfected with GFP-RNA or with RNA coding for a Mage3-DP4 specific TCR. As negative control target, unloaded autologous DC were used. As specific target, the Mage3-DP4 peptide was loaded on the DC. After 44 h the cytokine concentration in the supernatants was measured by CBA (A). Since IFNγ and IL-2 concentrations were out of scale (*) in one condition, the MFI of the beads is also depicted (B)

### MODES OF PRACTICING THE INVENTION

While the making and using of various embodiments of the present invention are discussed in detail below, it should be appreciated that the present invention provides many applicable inventive concepts that can be embodied in a wide variety of specific contexts. The specific embodiments discussed herein are merely illustrative of specific ways to make and use the invention and do not delimit the scope of the invention.

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims. For example, the term "a cell" includes a plurality of cells, including mixtures thereof. It also is to be understood, although not always explicitly stated, that the reagents described herein are merely exemplary and that equivalents of such are known in the art.

The activation state of a T cell defines whether the T cell is "resting" (i.e., in the Go phase of the cell cycle) or "activated" to proliferate after an appropriate stimulus such as the recognition of its specific antigen, or by stimulation with OKT3 antibody, PHA or PMA, etc. The "phenotype" of the T cell (e.g., naive, central memory, effector memory, lytic effectors, help effectors (T_{H}1 and T_{H}2 cells), and regulatory effectors), describes the function the cell exerts when activated. A healthy donor has T cells of each of these phenotypes, and which are predominately in the resting state. A naive T cell will proliferate upon activation, and then differentiate into a memory T cell or an effector T cell. It can then assume the resting state again, until it gets activated the next time, to exert its new function and may change its phenotype again. An effector T cell will divide upon activation and antigen-specific effector function.

The term "antigen" is well understood in the art and includes any molecule that can bind to an antibody, as well as epitopes, peptides fragments of antigens which can bind to MHC molecules, and immunogens. It will be appreciated that the use of any antigen is envisioned for use in the present invention and thus includes, but is not limited to a self-antigen (whether normal or disease-related), a tumor antigen, a pathogen antigen (e.g., a microbial antigen, viral antigen, etc.), or some other foreign antigen (e.g., a food component, pollen, etc.). T cell receptors bind to antigens or peptide fragments of antigens bound to MHC molecules. As used herein, a TCR receptor specific for an antigen includes T cell receptors specific for peptide fragments of the antigen.

The term "tumor associated antigen" or "TAA" refers to an antigen that is associated with a tumor. Examples of well known TAAs include survivin, gp100, MART, MAGE-1 and MAGE-3. Sequences of some peptides fragments of TAAs which bind MHC molecules include MAGE 1 nonapeptide (EADPTGHSY), MART-APL peptide (LAGIGILTV) or native peptide (AAGIGILTV) and PSA-1 peptide (FLTPKKLQCV). Sequences of additional tumor associated peptides and antigens are known to those of skill in the art.

The term "antigen presenting cells (APCs)" refers to a class of cells capable of presenting one or more antigens in the form of peptide-MHC complex recognizable by specific effector cells of the immune system, and thereby inducing an effective cellular immune response against the antigen or antigens being presented. APCs can be intact whole cells such as macrophages, B-cells, endothelial cells, activated T-cells, and dendritic cells; or other molecules, naturally occurring or synthetic, such as purified MHC Class I molecules complexed to β2-microglobulin. While many types of cells may be capable of presenting antigens on their cell surface for T-cell recognition, only dendritic cells have the capacity to present antigens in an efficient amount to activate naive T-cells for cytotoxic T-lymphocyte (CTL) responses.

By cancer or tumor is meant the abnormal presence of cells which exhibit relatively autonomous growth, so that a cancer cell exhibits an aberrant growth phenotype characterized by a significant loss of cell proliferation control. Cancerous cells can be benign or malignant. In various embodiments, the cancer affects cells of the bladder, blood, brain, breast, colon, digestive tract, lung, ovaries, pancreas, prostate gland, or skin. The definition of a cancer or tumor cell, as used herein, includes not only a primary cancer cell, but also any cell derived from a cancer cell ancestor. This includes metastasized cancer cells, and in vitro cultures and cell lines derived from cancer cells. Cancer or tumor includes, but is not limited to, solid tumors, liquid tumors, hematologic malignancies, renal cell cancer, melanoma, breast cancer, prostate cancer, testicular cancer, bladder cancer, ovarian cancer, cervical cancer, stomach cancer, esophageal cancer, pancreatic cancer, lung cancer, neuroblastoma, glioblastoma, retinoblastoma, leukemias, myelomas, lymphomas, hepatoma, adenomas, sarcomas, carcinomas, blastomas, etc.

"Co-stimulatory molecules" are involved in the interaction between receptor-ligand pairs expressed on the surface of antigen presenting cells and T cells. Research accumulated over the past several years has demonstrated convincingly that resting T cells require at least two signals for induction of cytokine gene expression and proliferation (Schwartz, R.H. (1990) Science 248: 1349-1356 and Jenkins, M.K. (1992) Immunol. Today 13:69-73). One signal, the one that confers specificity, can be produced by interaction of the TCR/CD3 complex with an appropriate MHC/peptide complex. The second signal is not antigen specific and is termed the "co-stimulatory" signal. This signal was originally defined as an activity provided by bone-marrow-derived accessory cells such as macrophages and dendritic cells, the so called "professional" APCs. Several molecules have been shown to enhance co-stimulatory activity. These are heat stable antigen (HSA) (Liu, Y. et al. (1992) 3. Exp. Med. 175:437-445), chondroitin sulfate-modified MHC invariant chain (li-CS) (Naujokas, M.F. et al. (1993) Cell 74:257-268), intracellular adhesion molecule 1 (ICAM-1) (Van Seventer, G.A. (1990). Immunol. 144:4579-4586), B7-1, and B7-2/B70 (Schwartz, R.H. (1992) Cell 71:1065-1068). These molecules each appear to assist co-stimulation by interacting with their cognate ligands on the T cells. Co-stimulatory molecules mediate co-stimulatory signal(s), which are necessary, under normal physiological conditions, to achieve full activation of naïve T cells. One exemplary receptor-ligand pair is the B7 family of co-stimulatory molecule on the surface of APCs and its counter receptor CD28 or CTLA-4 on T cells (Freeman, et al. (1993) Science 262:909-911; Young, et al. (1992). Clin. Invest. 90:229 and Nabavi, et al. (1992) Nature 360:266-268). Other important co-stimulatory molecules are CD40, and CD54. The term "costimulatory molecule" encompasses any single molecule or combination of molecules which, when acting together with a MHC/peptide complex bound by a TCR on the surface of a T cell, provides a co-stimulatory effect which achieves activation of the T cell that binds the peptide. The term thus encompasses B7, or other co-stimulatory molecule(s) on an antigen-presenting matrix such as an APC, fragments thereof (alone, complexed with another molecule(s), or as part of a fusion protein) which, together with MHC complex, binds to a cognate ligand and results in activation of the T cell when the TCR on the surface of the T cell specifically binds the peptide. It is intended, although not always explicitly stated, that molecules having similar biological activity as wild-type or purified co-stimulatory molecules (e.g., recombinantly produced or muteins thereof) are intended to be used within the spirit and scope of the invention.

The term "culturing" refers to the *in vitro* maintenance, differentiation, and/or propagation of cells in suitable media. By "enriched" is meant a composition comprising cells present in a greater percentage of total cells than is found in the tissues where they are present in an organism.

As used herein, the term "cytokine" refers to any one of the numerous factors that exert a variety of effects on cells, for example, inducing growth or proliferation. Non-limiting examples of cytokines which may be used alone or in combination in the practice of the present invention include, interleukin-2 (IL-2), stem cell factor (SCF), interleukin-3 (IL-3), interleukin-6 (IL-6), interleukin-12 (IL-12), G-CSF, granulocyte macrophage-colony stimulating factor (GM-CSF), interleukin-1 alpha (IL-1α), interleukin-1L (IL-11), MIP-11, leukemia inhibitory factor (LIF), c-kit ligand, thrombopoietin (TPO) and flt3 ligand. Cytokines are commercially available from several vendors such as, for example, Genzyme (Framingham, MA), Genentech (South San Francisco, CA), Amgen (Thousand Oaks, CA), R&D Systems (Minneapolis, MN) and Immunex (Seattle, WA). It is intended, although not always explicitly stated, that molecules having similar biological activity as wild-type or purified cytokines (e.g., recombinantly produced or muteins thereof) are intended to be used within the spirit and scope of the invention.

The term "dendritic cells (DCs)" refers to a diverse population of morphologically similar cell types found in a variety of lymphoid and non-lymphoid tissues (Steinman (1991) Ann. Rev. Immunol. 9:271-296). Dendritic cells constitute the most potent and preferred APCs in the organism. While the dendritic cells can be differentiated from monocytes, they possess distinct phenotypes. For example, a particular differentiating marker, CD14 antigen, is not found in dendritic cells but is possessed by monocytes. Also, mature dendritic cells are not phagocytic, whereas the monocytes are strongly phagocytosing cells. It has been shown that mature DCs can provide all the signals necessary for T cell activation and proliferation.

An "effective amount" is an amount sufficient to effect beneficial or desired results, such as enhanced immune response, treatment, prevention or amelioration of a medical condition (disease, infection, etc). An effective amount can be administered in one or more administrations, applications or dosages. Suitable dosages will vary depending on body weight, age, health, disease or condition to be treated and route of administration.

As used herein, "expression" refers to the processes by which in vitro transcribed (IVT) mRNA is translated into peptides, polypeptides, or proteins in a transfected cell. Regulatory elements required for expression include sequences for ribosome binding, translation initiation and a termination codon for detachment of the ribosome. Vectors for in vitro transcription of RNA can be obtained commercially or assembled by the sequences described in methods known in the art.

The term "genetically modified" means containing and/or expressing a foreign gene or nucleic acid sequence which in turn, modifies the genotype or phenotype of the cell and its progeny. In other words, it refers to any addition, deletion or disruption to a cell's endogenous nucleotides. For example, retroviral transduction results in genetic modification of a cell's genome. In contrast, transient transfection with mRNA does not result in genetic modification.

The term "effector T cells", as used herein, refers to T cells that can specifically bind an antigen and mediate an immune response (effector function) without the need for further differentiation. Examples of effector T cells include CTLs, T_{H}1 cells, T_{H}2 cell and regulatory T cells (T_{regs}). In contrast to effector T cells, naïve T cells have not encountered their specific antigen:MHC complex, nor responded it to it by proliferation and differentiation into an effector T cell. Effector T cells can be resting (in the Go phase of the cell cycle) or activated (proliferating).

"Immune response" broadly refers to the antigen-specific responses of lymphocytes to foreign or self substances. Any substance that can elicit an immune response is said to be "immunogenic" and is referred to as an "immunogen". All immunogens are antigens, however, not all antigens are immunogenic. Immune responses include humoral responses (via antibody activity) and cell-mediated responses (via T cell activation).

The term "isolated" means separated from constituents, cellular and otherwise, in which the polynucleotide, peptide, polypeptide, protein, antibody, or fragments thereof, are normally associated with in nature. For example, with respect to a polynucleotide, an isolated polynucleotide is one that is separated from the 5' and 3' sequences with which it is normally associated in the chromosome. As is apparent to those of skill in the art, a non-naturally occurring polynucleotide, peptide, polypeptide, protein, antibody, or fragment(s) thereof, does not require "isolation" to distinguish it from its naturally occurring counterpart. In addition, a "concentrated", "separated" or "diluted" polynucleotide, peptide, polypeptide, protein, antibody, or fragment(s) thereof, is distinguishable from its naturally occurring counterpart in that the concentration or number of molecules per volume is greater than "concentrated" or less than "separated" than that of its naturally occurring counterpart. A polynucleotide, peptide, polypeptide, protein, antibody, or fragment(s) thereof, which differs from the naturally occurring counterpart in its primary sequence or for example, by its glycosylation pattern, need not be present in its isolated form since it is distinguishable from its naturally occurring counterpart by its primary sequence, or alternatively, by another characteristic such as its glycosylation pattern. A mammalian cell, such as T-cell, is isolated if it is removed from the anatomical site from which it is found in an organism.

The terms "major histocompatibility complex" or "MHC" refers to a complex of genes encoding cell-surface molecules that are required for antigen presentation to T cells and for rapid graft rejection. In humans, the MHC is also known as the "human leukocyte antigen" or "HLA" complex. The proteins encoded by the MHC are known as "MHC molecules" and are classified into Class I and Class II MHC molecules. Class I MHC molecules include membrane heterodimeric proteins made up of an α chain encoded in the MHC noncovalently linked with the β₂-microglobulin. Class I MHC molecules are expressed by nearly all nucleated cells and have been shown to function in antigen presentation to CD8⁺ T cells. Class I molecules include HLA-A, B, and C in humans. Class II MHC molecules also include membrane heterodimeric proteins consisting of noncovalently associated α and β chains. Class II MHC molecules are known to function in CD4⁺ T cells and, in humans, include HLA-DP, -DQ, and -DR.

"Pathogen", as used herein, refers to any disease causing organism or virus, and also to attenuated derivatives thereof. The term pathogen refers to any virus or organism which is involved in the etiology of a disease and also to attenuated derivatives thereof. Such pathogens include, but are not limited to, bacterial, protozoan, fungal and viral pathogens such as Helicobacter, such as Helicobacter pylori, Salmonella, Shigella, Enterobacter, Campylobacter, various mycobacteria, such as Mycobacterium leprae, Mycobacterium tuberculosis, Bacillus anthracis, Yersinia pestis, Francisella tularensis, Brucella species, Leptospira interrogans, Staphylococcus, such as S. aureus, Streptococcus, Clostridum, Candida albicans, Plasmodium, Leishmania, Trypanosoma, human immunodeficiency virus (HIV), hepatitis C virus (HCV), human papilloma virus (HPV), cytomegalovius (CMV), HTLV, herpes virus (e.g., herpes simplex virus type 1, herpes simplex virus type 2, coronavirus, varicella-zoster virus, and Epstein-Barr virus), papilloma virus, influenza virus, hepatitis B virus, poliomyelitis virus, measles virus, mumps virus, and rubella virus.

The term "peptide" is used in its broadest sense to refer to a compound of two or more subunit amino acids, amino acid analogs, or peptidomimetics. The subunits may be linked by peptide bonds. In another embodiment, the subunit may be linked by other bonds, e.g., ester, ether, etc. As used herein the term "amino acid" refers to either natural and/or unnatural or synthetic amino acids, including glycine and both the D and L optical isomers, amino acid analogs and peptidomimetics. A peptide of three or more amino acids is commonly called an oligopeptide if the peptide chain is short. If the peptide chain is long, the peptide is commonly called a polypeptide or a protein.

The terms "polynucleotide", "nucleic acid" and "nucleic acid molecule" are used interchangeably to refer to polymeric forms of nucleotides of any length. The polynucleotides may contain deoxyribonucleotides, ribonucleotides, and/or their analogs. Nucleotides may have any three-dimensional structure, and may perform any function, known or unknown. The term "polynucleotide" includes, for example, single-stranded, double-stranded and triple helical molecules, a gene or gene fragment, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes, and primers. In addition to a native nucleic acid molecule, a nucleic acid molecule of the present invention may also comprise modified nucleic acid molecules.

The term "RNA" refers to polymeric forms of ribonucleotides of any length, wherein the ribonucleotides or ribonucleotide analogs are joined together by phosphodiester bonds. The term "RNA" includes, for example, single-stranded, double-stranded and triple helical molecules, primary transcripts, mRNA, tRNA, rRNA, in vitro transcripts, in vitro synthesized RNA, branched polyribonucleotides, isolated RNA of any sequence, and the like. mRNA refers to an RNA that can be translated in a cell. Such mRNAs typically are capped and have a ribosome binding site (Kozak sequence) and a translational initiation codon. For example, in one aspect, the invention relates to the transfection of T cells with mRNA that can be translated in the transfected T cell.

A "pharmaceutical composition" is intended to include the combination of an active agent with a carrier, inert or active, making the composition suitable for diagnostic or therapeutic use in vitro, in vivo or ex vivo.

As used herein, the term "pharmaceutically acceptable carrier" encompasses any of the pharmaceutical carriers compatible with T cells, such as a phosphate buffered saline solution, protein excipients include serum albumin such as human serum albumin (HSA), recombinant human albumin (rHA), gelatin, casein, and the like. For examples of carriers, stabilizers and adjuvants, see Martin REMINGTON'S PHARM. SCI., 18th Ed. (Mack Publ. Co., Easton (1995)) and the "PHYSICIAN'S DESK REFERENCE", 58nd Ed., Medical Economics, Montvale, N.J. (2004). The term carrier can include a buffer or a pH adjusting agent; typically, the buffer is a salt prepared from an organic acid or base. Representative buffers include organic acid salts such as salts of citric acid, ascorbic acid, gluconic acid, carbonic acid, tartaric acid, succinic acid, acetic acid, or phthalic acid; Tris, tromethamine hydrochloride, or phosphate buffers. Additional carriers include polymeric excipients/additives such as polyvinylpyrrolidones, ficolls (a polymeric sugar), dextrates (e.g., cyclodextrins, such as 2-hydroxypropyl-.quadrature.-cyclodextrin), polyethylene glycols, antioxidants, antistatic agents, surfactants (e.g., polysorbates such as "TWEEN 20" and "TWEEN 80"), lipids (e.g., phospholipids, fatty acids), steroids (e.g., cholesterol), and chelating agents (e.g., EDTA). Agents which prevent or reduce ice formation, may be included.

Effective transfer of TCR function in T cells was previously only possible by stable transduction using a retroviral vector encoding a TCR. However, retroviral transduction poses the threat of irreversible genetic manipulation of autologous cells. We have developed optimized conditions for transient RNA transfection of T cells. Transfection efficiency, using EGFP-RNA and the optimized conditions, was >90%. The electroporation of primary T cells, isolated from blood, with TCR-coding RNA resulted in functional CTLs (>60% killing at an effector:target ratio of 20:1) with the same HLA-A2/gp100-specificity as the parental CTL clone. The TCR-transfected T cells specifically recognized peptide-pulsed T2 cells, or dendritic cells electroporated with gp100-coding RNA, in an IFNγ-secretion assay and retained this ability, even after cryopreservation, for over 3 days. Importantly, this is the first time that the CD8+ T cells electroporated with TCR RNA displayed cytotoxicity, and specifically lysed peptide-loaded T2 cells and HLA-A2⁺/gp100⁺ melanoma cells over a period of at least 72h. To our knowledge, this is the first description of the transfer of *cytolytic* capacity by TCR-RNA transfection into T cells. Peptide-titration studies showed that the lytic efficiency of the RNA-transfected T cells was similar to that of retrovirally transduced T cells, and approximated that of the parental CTL clone. The ability of cryopreserved TCR RNA-electroporated CD8+ T cells to produce IFNγ and exert cytotoxicity is essential for the generation of large batches of clinically feasible and effective transfected T cells for immunotherapy.

Since this strategy overcomes disadvantages of retroviral transduction (e.g., the possible insertional mutagenesis), it provides a new and better way to generate antigen-specific T lymphocytes for the immunotherapy of cancer, pathogen infection and the treatment of autoimmune diseases, as well as transplant rejection. The problem of possible insertional mutagenesis is completely absent when RNA is electroporated into cells, and for applications where only transient expression of certain molecules is necessary, RNA electroporation is a good alternative. Since RNA is only transiently present in the transfected cell, and is not integrated into the genome, this procedure cannot be classified as gene therapy. Therefore, our TCR RNA transfection of T cells is much safer than retroviral transduction of TCR genes into T cells. Another important aspect is that, due to the safety and simplicity of the RNA transfection (both from a technical and a regulatory point of view), a rapid screening of candidate TCRs for therapeutic usefulness is possible. Apart from these advantages of TCR RNA-transfected T cells over retrovirally transduced T cells for the use in adoptive transfer, TCR RNA-transfected T cells can be used as "reagents" and substitutes for reportedly unstable human T cell clones, to detect and monitor specific MHC/peptide complexes on APC and target cells *in vitro.* The possibility to cryopreserve several batches of one preparation allows a supply of constant quality over long term experiments.

While it is possible to electroporate large numbers of T cells, this is not necessary when transient transfected T cells are injected in a tumor. Few T cells can cause destruction of parts of the tumor, which subsequently may cause epitope spreading by effective presentation of antigens by antigen presenting cells. It is also possible to use a combination-therapy, first injecting TCR-transfected T cells to induce epitope-spreading, and then injecting dendritic cells, either unloaded or pre-loaded with antigen. In these cases, many T cells and/or long-term expression of the specific TCR are not needed. In addition TCR transfected CD4+ cells can provide T cell help for tumor rejection.

Electroporation of RNA coding for the full length TCR α and β (or γ and δ) chains can be used as alternative to overcome long-term problems with autoreactivity caused by pairing of retrovirally transduced and endogenous TCR chains. Even if such alternative pairing takes place in the transient transfection strategy, the possibly generated autoreactive T cells will loose this autoreactivity after some time, because the introduced TCR α and β chain are only transiently expressed. When the introduced TCR α and β chain expression is diminished, only normal autologous T cells are left. This is not the case when full length TCR chains are introduced by stable retroviral transduction, which will never loose the introduced TCR chains, causing a constantly present autoreactivity in the patient.

Some success was previously reported in electroporating T-cells with green fluorescent protein (GFP). However, both the GFP RNA and protein are highly stable, and transfection efficiencies observed with other RNAs are typically lower than those achieved with GFP RNA. Prior to the instant invention, it was not thought possible to achieve a high enough efficiency of transfection of T cells with RNA encoding a membrane protein, and particularly of a heterodimeric membrane protein, to achieve functional expression. The present invention provides optimized methods for electroporating T cells with RNA, which allow for the functional expression of TCR RNA and other RNAs in transiently transfected T cells. The methods disclosed herein may be used to transfect any type of T cell with RNA. In preferred embodiments, the RNA encodes a TCR. Expression of the TCR RNA in the T cell results in antigen-specific effector function in response to ligation of the TCR with antigen:MHC complex.

Accordingly, the invention provides a composition comprising an effector T cell transiently transfected with RNA encoding a T cell receptor (TCR) specific for an antigen, wherein the T cell has effector function specific for cells presenting the antigen in complex with an MHC molecule.

"T cell" refers to T lymphocytes, and includes, but is not limited to, γ:δ⁺ T cells, NK T cells, CD4+ T cells and CD8+ T cells. CD4+ T cells include T_{H}0, T_{H}1 and T_{H}2 cells, as well as regulatory T cells (T_{reg}). There are at least three types of regulatory T cells: CD4+ CD25+ T_{reg}, CD25⁻ T_{H}3 T_{reg}, and CD25⁻ T_{R}1 T_{reg}. "Cytotoxic T cell" refers to a T cell that can kill another cell. The majority of cytotoxic T cells are CD8+ MHC class I-restricted T cells, however some cytotoxic T cells are CD4+. Any type of T cell can be transfected using the methods herein. In preferred embodiments, the T cell is CD4+ or CD8+. In one embodiment, the T cell is a T_{reg} cell.

Most T cell receptors (TCRs) recognize the complex of a peptide antigen (or a peptide fragment of an antigen) bound to an MHC molecule (MHC:antigen complex). The TCR is responsible for the antigen specificity of each T cell, as well as for restriction to recognition of antigen displayed by MHC class I molecules versus MHC class II molecules. TCRs originating in CD4+ T cells are MHC class II restricted, meaning that TCRs originating from CD4+ T cells only recognize antigen displayed by MHC class II molecules. TCRs originating from CD8+ T cells are MHC class I restricted, and only recognize antigen displayed by MHC class I molecules. In one embodiment, a CD8+ T cell is transfected with RNA encoding one or more MHC class I restricted TCRs. In another embodiment, a CD4+ T cell is tranfected with RNA encoding one or more MHC class II restricted TCRs.

Surprisingly, the inventors have discovered that MHC class I specific antigen recognition can be imparted to a CD4+ T cell by transfection with a nucleic acid encoding an MHC class I specific TCR. Similarly, MHC class II specific antigen recognition can be imparted to a CD8+ T cell by transfection with a nucleic acid encoding an MHC class II specific TCR. As used herein, a TCR is considered "specific" for a particular antigen if a T cell that carries this TCR exerts immunological function (such as the release of cytokines, lysis of the stimulator cell, etc.) significantly (p<0.05) better when stimulated with that antigen (in complex with an MHC molecule) than when stimulated with an unrelated antigen. Thus, in one embodiment, a CD8+ T cell is transfected with a nucleic acid, preferably an RNA, encoding one or more MHC class II restricted TCRs. In other embodiment, a CD4+ T cell is transfected with a nucleic acid, preferably an RNA, encoding one or more MHC class I restricted TCRs. In addition, CD8+ T cells can be transfected with nucleic acids, preferably RNA, encoding both MHC class I and MHC class II restricted TCRs. Similarly, CD4+ T cells can be transfected with nucleic acids, preferably RNA, encoding both MHC class I and MHC class II restricted TCRs. TCRs useful in the invention also include chimeric non-MHC-restricted TCRs that can recognize antigen whether or not the antigen is complexed with an MHC molecule. A non-MHC-restricted TCR is considered "specific" for a particular antigen if a T cell that carries this TCR exerts immunological function (such as the release of cytokines, lysis of the stimulator cell, etc.) significantly (p<0.05) better when stimulated with that antigen (whether or not the antigen is in complex with an MHC molecule) than when stimulated with an unrelated antigen. Accordingly, both CD4+ T cells and CD8+ T cells can be transfected with nucleic acids, preferably RNA encoding non-MHC restricted TCRs, alone or in combination with MHC restricted TCRs.

Naturally occurring TCRs are heterodimeric glycoproteins composed of two polypeptide chains, either alpha and beta chains (TCRα and TCRβ) or gamma and delta chains (TCRγ and TCRδ). α:β TCRs are naturally expressed in CD8+ T cells and CD4+ T cells, while γ:δ TCRs are naturally expressed in a subset of T cells termed γ:δ⁺ T cells. TCR diversity is generated by a series of rearrangements of variable region gene segments during the development of the T cell in the thymus. Each chain has an extracellular variable region, an extracellular constant region, a hinge region with a cysteine residue for forming a disulfide linkage between the two chains, a transmembrane region and a cytoplasmic tail. The two variable regions of the heterodimer form a single antigen binding site. The complementarity determining region (CDR) 3 of the variable regions of the alpha and beta TCR chains interact with peptide. The CDR 1 and 2 regions of the variable regions of the alpha and beta TCR chains interact with the MHC molecule.

The invention provides T cells transiently transfected with RNA encoding a T cell receptor specific for an antigen. The TCR can be MHC class I restricted, MHC class II restricted, or non-MHC restricted (MHC independent). Each of these types of TCRs are known to those of skill in the art. Examples of non-MHC-restricted chimeric receptors are disclosed in Bolhuis et al. Adv Exp Med Biol. 1998;451:547-55; Weijtens et al. Gene Ther. 1998 Sep;5(9):1195-203; Weijtens et al. Int J Cancer. 1998 Jul 17;77(2):181-7; Eshhar et al. J Immunol Methods. 2001 Feb 1;248(1-2):67-76; Hombach et al. Int J Cancer. 2000 Oct 1;88(1):115-20; and Daly et al. Cancer Gene Ther. 2000 Feb;7(2):284-91; the contents of which are incorporated by reference. Both CD4+ and CD8+ T cells may be transfected with any type of TCR. Also, the T cells may be cotransfected with additional RNAs encoding other polypeptides of interest, such as cytokines, transcriptional regulators (e.g., FoxP3), costimulatory molecules, etc. However, as used herein, transfection with an RNA encoding a T cell receptor excludes transfection with the total RNA or total mRNA of a T-cell or T cell derivative (such as a T cell tumor), unless the proportion of RNA encoding a TCR is enriched with respect to it's normal representation in the total RNA or total mRNA.

By "RNA encoding a T cell receptor" is meant one or more RNAs that encode a functional T cell receptor, in that the expressed T cell receptor can specifically bind the antigen it normally recognizes, when the antigen is complexed with an MHC molecule of the class recognized by the TCR (or in the absence of a complex with an MHC molecule if the TCR is a non-MHC restricted TCR). In most cases, this RNA will include an RNA encoding an alpha chain of a TCR and an RNA encoding the corresponding beta chain of the TCR (or alternatively an RNA encoding a delta chain of a TCR and an RNA encoding the corresponding gamma chain of a TCR). Alternatively a chimeric receptor can be used, which would avoid mispairing and could circumvent HLA restriction. A chimeric TCR polypeptide can be generated by fusing domains from different proteins together. For example, the intracellular domain would typically include an intracellular signaling domain of the TCR-complex, for example the CD3 zeta-chain, or a signaling domain that functions in a similar fashion, for example signaling domains from Fc receptors. Extracellular domains can be chosen that are capable of specific binding to antigen in an MHC context, for example the extracellular domains of TCR alpha and beta chains, or extracellular domain capable of binding antigen independent of MHC, for example an antigen specific scFv. The transmembrane domain can be taken or derived from the proteins, from which either the intracellular domain or the extracellular domain was also taken, or from other transmembrane proteins.

In most studies in which TCR genes were retrovirally transduced into T cells, full length TCR α and β chain genes were used to retarget these T cells. However, there is a potential hazard in using full length TCR chain genes, because in theory, the introduced chains can pair with the endogenous TCR chains [8, 30, 32]. This alternative pairing can lead to unpredictable specificities, which may be autoreactive. This formation of self-reactive TCRs is a recognized concern for gene therapy regulatory committees. That alternative pairing takes place was shown in studies in which full length TCR chains specific for gp100 or MDM-2 were retrovirally transferred to T cells. Only a fraction of the T cells expressing the introduced TCR β chain (i.e. 50-60% and 30-50%, respectively) were able to bind the respective MHC/peptide tetramer [24, 27].

One solution for the alternative-pairing problem is to introduce single or two chain modified TCR-based receptors, that are structurally different from full length TCRs, resulting in exclusive pairing between the introduced TCR chains [8]. Methods for constructing single chain TCRs (scTCR) are disclosed in Lake et al. (1999) Int Immunol 11:745-751 and Nitta et al. (1990) Science 249:672, the contents of which are incorporated by reference. Such receptors specific for several melanoma antigens, e.g. MAGE-1 [29], gp100 [23], have been functionally introduced in T cells by retroviral transduction. However, careful comparison of full length and modified single chain TCR specific for an OVA peptide showed that single chain TCR-transduced T cells were less efficient in response to stimulation with OVA peptide-pulsed target cells (i.e. especially when low concentrations of peptide were used) and natively expressing target cells as compared to full length TCR-transduced T cells [32]. Therefore, to generate the most effective T cells, it is preferable to use full length TCR chains for TCR transfer.

Preferably, the TCR chains are from mammals, more preferably from primates, and most preferably from humans. In preferred embodiments, the TCR specifically recognizes an antigen from a tumor or a pathogen, or a self-antigen. Preferably, the antigen is a tumor-specific or a pathogen specific antigen. Preferred tumor antigens include those from the following types of tumors: renal cell carcinoma, melanoma, chronic lymphocytic leukemia, breast cancer, lung cancer, prostate cancer, ovarian cancer and colon cancer. Examples of tumor specific antigens include, but are not limited to, MART-1, MAGE-1, MAGE-3 gp75, MDM2, tyrosinase, telomerase, gp100, survivin, alpha-1 fetoprotein, G250 and NY-ESO-1. Preferred pathogen antigens include antigens from HIV and HCV.

The sequences of numerous alpha, beta, gamma and delta TCR chains are known in the art (see, for example, Arden et al. (1995) Immunogenetics 42:455-500, the contents of which is incorporated by reference). GenBank currently contains more than 12,000 entries for T cell receptor sequences of various vertebrate species. Methods of making and screening TCR libraries are disclosed in U.S. patent publication 2003/0082719, the contents of which are incorporated by reference. Methods for cloning additional TCR chains is are known to those of skill in the art. For example, TCR chains can be cloned by identifying a T cell which expresses a TCR of the desired specificity, and reversely transcribing its RNA into DNA. The subtypes of the TCRα and β chains can then be determined by PCR. Identification of the subtype allows the selection of specific primers that can amplify the full length coding sequences of both chains. Methods for using PCR to determine the subtypes of the TCRα and β chains, and to choose primers for RT-PCR amplification of TCR chains are disclosed in Lake et al. (1999) Int Immunol 11:745-751 and Nitta et al. (1990) Science 249:672, the contents of which are incorporated by reference. Methods and primers for cloning TCRγ and δ chains by RT-PCR are disclosed in Kapp et al. (2004) Immunology 111:155-164; Weber-Arden et al. J Immunol Methods. 1996 197(1-2):187-92; and Olive (1995) Neuroimmunol 62:1-7, the contents of which are incorporated by reference. An alternative to the above method beginning with identification of the subtypes of the TCRα and β chain, would be to instead reversely transcribe a cDNA copy of the TCR RNA using a primer for reverse transcription which hybridizes to a conserved region of the TCR RNA, and then attach a defined sequence to the 3' end of the cDNA (e.g., by terminal transferase or by the improved capswitch technique (see WO 2005/052128, the contents of which are incorporated by reference). Methods for RT-PCR of RNA extracted from any cell (e.g., T cell), and in vitro transcription are disclosed in copending applications WO 2005/052128 and PCT/US05/32710, the contents of which are incorporated by reference.

cDNA copies of TCR RNAs can be inserted into an expression cassette for in vitro transcription. Alternatively, TCR cDNAs can be amplified using primers containing transcription and translation signals appropriate for in vitro transcription, as well as for translation of the in vitro transcribed (IVT) RNA within electroporated T cells. To ensure optimal translation in T cells, the IVT RNA encoding the TCR chain is preferably capped and polyadenylated. Also, the stability and/or translational efficiency of the mRNA can be increased by incorporating additional noncoding sequences, such as 5' and 3' UTRs.

Methods of *in vitro* transcription are known to those skilled in the art (see, for example, U.S. 2003/0194759, the content of which is incorporated by reference). In typical *in vitro* transcription reactions, a DNA template is transcribed using a bacteriophage RNA polymerase in the presence of all four ribonucleoside triphosphates and a cap dinucleotide such as m⁷G(5')ppp(5')G or a cap analog, such as ARCA. Such methods are routine in the art and are disclosed in the following publications: Sambrook et al. MOLECULAR CLONING: A LABORATORY MANUAL, 2nd edition (1989); CURRENT PROTOCOLS IN MOLECULAR BIOLOGY (Ausubel et al. eds. (1987)); the series METHODS IN ENZYMOLOGY (Academic Press, Inc.); PCR: A PRACTICAL APPROACH (M. MacPherson et al. IRL Press at Oxford University Press (1991)); and PCR 2: A PRACTICAL APPROACH (MacPherson, Hames and Taylor eds. (1995)).

Transient transfection of T cells with TCR IVT mRNA results in translation of the RNA and localization of the TCR heterodimer (or chimeric TCR) in the cell membrane. Binding of this TCR to the antigen in complex with an MHC molecule results in effector function by activated T cells. By transiently transfected is meant that the RNA transfected into the cell does not integrate into the host genome or replicate independently within the cell. In contrast retroviral transduction relies on integration of the retroviral vector into the host chromosome.

In a preferred embodiment, resting T cells, preferably resting effector T cells, are electroporated with RNA, preferably RNA encoding a TCR, and/or FoxP3 RNA. Thymocytes differentiate into mature T cells in the thymus and emigrate to the bloodstream and periphery with a naive phenotype and in a resting state. When they encounter their specific antigen:MHC complex together with costimulatory signals they get activated, start dividing, and acquire a new phenotype, for example that of an effector T cell or central memory T cell. They can enter a resting stage again if the antigen is removed. When the antigen:MHC complex is encountered again, no costimulation is required, and the T cell gets rapidly activated again. It will again divide and a memory T cell can differentiate further into an effector T cell. But even then it can become resting again. Peripheral T cells from a healthy donor usually comprise a mixture of naive, memory and effector T cells, the majority of which are in a resting stage. These resting T cells infrequently divide and are generally small with condensed chromatin and little cytoplasm. Upon activation, they rapidly proliferate and increase in size. Resting T cells can also be activated in vitro by stimulation with PHA, PMA or OKT3, typically in the presence of IL-2. Surprisingly, the inventors have discovered that electroporation of resting T cells with TCR RNA imparts effector function specific to the transfected TCR without the need for stimulation with PHA. PMA or OKT3 Ab.

Resting CD4+ and CD8 T cells are typically CD25- HLA DR⁻ and L-selectin⁺, and do not divide or express cytokines. Activated T cells are typically L-selectin⁻, CD25⁺, HLA DR⁺, divide rapidly and produce a variety of cytokines including IL2, IFNy and TNF. Methods for isolating T cells from peripheral blood are known to those of skill in the art, and are also described herein.

T-cell effector function can include, but is not limited to, one or more of IL-2 secretion, Tumor Necrosis Factor (TNF-α) secretion, interferon-γ (IFNγ) secretion, cytotoxicity, helper function (e.g., activation of macrophages and/or activation of B cells) and regulatory function. The effector function depends upon the type of T cell that was transfected. The effector functions of activated CD8+ T cells include cytotoxicity and IFNγ secretion. The effector function of activated CD4+ T_{H}1 cells includes activation of macrophages. The effector function of activated CD4+ T_{H}2 cells includes activation of B cells to proliferate and produce antibodies. Regulatory effector function includes, but is not limited to IL-10 secretion and/or TGF-β secretion. Methods of detecting and measuring effector function are known to those of skill in the art.

The effector function of T cells are determined by the effector molecules that they release in response to specific binding of their TCR with antigen:MHC complex on the target cell. Cytotoxic effector molecules stored in lytic granules that can be released by cytotoxic CD8+ T cells include perforin, granzymes, granulysin and Fas ligand. Perforin forms transmembrane pores in the target cell. Granzymes are serine proteases which can trigger apoptosis. Granulysin induces apoptosis in the target cells. Fas ligand can also induce apoptosis in target cells. Other effector molecules that can be released by cytotoxic T cells include IFN-γ, TNF-β and TNF-α. IFN-γ inhibits viral replication and activates macrophages. TNF-β and TNF-α can participate in macrophage activation and in killing some target cells. Transfection of a CD8+ T cell with TCR RNA derived from a CD8+ T cell results in MHC class I restricted antigen-specific cytotoxicity (i.e., cytotoxicity towards target cells which display the antigen:MHC class I complex). In contrast, transfection of a CD8+ T cell with TCR RNA derived from a CD4+ T cell results in MHC class II restricted antigen-specific cytotoxicity (i.e., cytotoxicity towards target cells which display the antigen:MHC class II complex). Antigens from intracellular pathogens (e.g., mycobacteria, the causative agent of tuberculosis and leprosy) are typically displayed on MHC class II molecules. Accordingly, transfection of a CD8+ T cell with RNA encoding a TCR specific for a mycobacteria antigen can result in MHC class II restricted antigen specific cytotoxicity towards mycoplasma infected cells.

Macrophage activating effector molecules that can be secreted by CD4+ T_{H}1 cells include IFN-γ, TNF-α, GM-CSF, CD40 ligand (CD154) and Fas ligand. A subset of CD4+ T_{H}1 cells can also assist in B-cell activation IFN-γ and CD40 ligand activate macrophages to destroy engulfed bacteria. Other effector molecules that can be released by T_{H}1 cells include IL-3, TNF-β (which inhibits B-cells), IL-2, CXCL2 and GROβ. Fas ligand and TNF-β can kill cell chronically infected with intracellular bacteria. IL-2 induces T cell proliferation. IL-3 and GM-CSF induces macrophage differentiation. CCL2 induces chemotaxis of macrophages. Transfection of a CD4+ T_{H}1 cell with TCR RNA derived from a CD4+ T cell results in MHC class II restricted effector function (i.e., macrophage activation in response to antigen-specific binding of target cells which display the antigen:MHC class II complex). In contrast, transfection of a CD4+ T_{H}1 cell with TCR RNA derived from a CD8+ T cell results in MHC class I restricted effector function (i.e., high IL-2, TNF and IFN secretion, macrophage activation in response to antigen-specific binding to target cells which display the antigen:MHC class I complex).

B-cell activating effector molecules that can be secreted by CD4+ T_{H}2 cells include IL-4, IL-5, IL-9, IL-13 IL-15 and CD40 ligand. Other effector molecules that can be released by T_{H}2 cells include IL-3, GM-CSF, IL-10 (which inhibits macrophage activation), TGF-β, IL-2, CCL11 (eotaxin) and CCL17 (TARC). Activated TH2 cells (and some TH1 cells) stimulate B cells to proliferate and differentiate when they recognize a specific antigen:MHC class II complex displayed by a B cell. Transfection of a CD4+ T_{H}2 cell with TCR RNA derived from a CD4+ T cell results in MHC class II restricted effector function (i.e., B-cell activation in response to antigen-specific binding of target cells which display the antigen:MHC class II complex). In contrast, transfection of a CD4+ T_{H}1 cell with TCR RNA derived from a CD8+ T cell results in MHC class I restricted effector function (i.e., B cell activation in response to antigen-specific binding to target cells which display the antigen:MHC class I complex).

CD4+ regulatory T cells down-regulate the immune response. T_{R}1 regulatory T cells secrete immunosuppressive effector molecules, such as IL-10 and TGF-β. IL-10 down-regulates T-cell responses by reducing the production of IL-2, TNF-α and IL-5 by T-cells. TGF-β decreases T-cell proliferation, killing and cytokine expression. T_{H}3 regulatory T cells down-regulate the immune response by the secretion of the immunosuppressive effector molecule TGF-β. CD4+ CD25+ regulatory T cells are immunosuppressive, and are activated by antigen-specific binding to their TCR. Once activated, CD4+ CD25+ regulatory T cells exhibit immunosuppressive effector function in an antigen-independent manner.

Transiently transfected effector T cells of can be used to treat tumors, pathogen infection, autoimmune disease, GVHD, and to prevent transplant rejection. In preferred embodiments of the invention, the TCR is specific for a tumor antigen, a pathogen antigen or a self-antigen. Preferably, the antigen is a tumor antigen or a pathogen antigen. The antigen can be from any type of tumor, including, but not limited to renal cell carcinoma, melanoma, chronic lymphocytic leukemia, breast cancer, lung cancer, prostate cancer, ovarian cancer or colon cancer. Preferred melanoma antigens include MART-1, MAGE-1, MART-1, and gp100. Other preferred tumor antigens include gp75, MDM2, tyrosinase, telomerase, survivin, alpha 1 fetoprotein, CA125, CA15-3, CA19-9, PSA, G250 and NY-ESO-1. Additional tumor associated antigens and methods for their identification of TAAs are disclosed in Nicolette and Miller (2003) Drug Discovery Today 8:31-38; Kawakami and Rosenberg (1997) Immunol Res 16:313 and Slingluff et al. (1994) Curr Opin Immunol 6:733, the contents of which are incorporated by reference. Preferred pathogen antigens are HIV and HCV antigens. In cancer therapy, one source for tumor specific TCRs would be the tumor infiltrating lymphocytes. Although anergic they express functional TCRs. Another possibility would be the in vitro stimulation of patient derived T cells. In the absence of regulatory mechanisms, TAA specific T cells can be expanded. Once an array of TCRs is generated, an assortment out of these can individually be chosen for each patient, with respect to MHC type and the antigen expression of the tumor. In HIV therapy, some immunogenic peptides are well characterized. However, the immune system of most patients is already too weak to mount an efficient immune response against the virus. TCRs could be generated from HIV infected patients exhibiting an effective immune response (e.g. early on or later in long-term non-progressors) or in healthy donors that participated in vaccination trials.

As described above, RNA encoding either an MHC class I restricted TCR, an MHC class II restricted TCR and/or a non-MHC restricted TCR can be transferred into any type of T cell by electroporation. In one embodiment, the T cell is a regulatory T cell (T_{reg}). Preferably, the T_{reg} is CD4+CD25+. Regulatory T cells transfected with TCR RNA specific for a self-antigen are useful for the treatment of autoimmune disease. Regulatory T cells transfected with TCR RNA specific for a transplant antigen can be useful to prevent transplant rejection and to treat or prevent graft vs. host disease (GVHD).

FoxP3 is a transcription factor that is involved in the differentiation of CD4+ T cells into regulatory T cells. Retroviral expression of FoxP3 is sufficient to convert CD4+ T cells into regulatory T cells (Sakaguchi et al. (2003) Science 299:1057-61). Thus, in one embodiment, the invention provides a T_{reg} cell comprising an exogenous RNA encoding FoxP3. The human Foxp3 amino acid sequence and cDNA sequence are disclosed in GenBank accession number NM_014009 (VERSION NM_014009.2 GI:31982942). By exogenous RNA is meant an RNA introduced directly by transfection with RNA, or by transcription of an exogenous expression cassette. Accordingly, CD4+ T cells can be transfected with an RNA encoding FoxP3, or with an expression cassette for FoxP3. In preferred embodiments, the T cell is transiently transfected with RNA encoding FoxP3. Also, T cells can be cotransfected with FoxP3 RNA and TCR RNA.

The inventors have optimized methods for electroporating T cells with RNA. Preferably, purified CD8+ T cells or purified CD4+ T cells are electroporated. Electroporation of a T cell with an RNA encoding a TCR imparts new antigen-specificity to the transfected T cell. Accordingly, in one aspect, the invention provides a method for imparting a new antigen specificity to a T cell, comprising electroporating a composition comprising purified CD8+ or purified CD4+ T cells with RNA encoding a TCR receptor specific for an antigen.

By purified CD8+ T cells is meant that the ratio of CD8⁺ T cells:CD8- T cells in a purified CD8+ T cell composition is increased in comparison to the ratio of CD8⁺ T cells:CD8- T cells in peripheral blood. Similarly, by purified CD4+ T cells is meant that the ratio of CD4⁺ T cells:CD4- T cells in a purified CD4+ T cell composition is increased in comparison to the ratio of CD8⁺ T cells:CD8- T cells in peripheral blood. Preferably, the purified T cells (CD8+ or CD4+) comprise at least 75%, more preferably at least 90% and most preferably at least 95% or even at least 99% of all T cells present in the composition. Methods for purifying CD4+ or CD8+ T cells are known to those of skill in the art. In a preferred embodiment, the T cells are purified by magnetic sorting. In one embodiment, T_{regs} are separated or removed from CD8+ T cells or CD4+ T helper cells.

Prior to electroporation, the T cells can be unstimulated (and predominately resting) or stimulated in vitro, (e.g., by OKT3 Ab, PHA, PMA, etc.). Preferably, the purified CD8+ T cells or CD4+ have not been stimulated in vitro by phytohemaglutinin (PHA) or OKT3 prior to electroporation. Thus, in another aspect, the invention provides a method for imparting a new antigen specificity to T cells, comprising: electroporating resting T cells with RNA encoding a TCR specific for an antigen.

However, cases where it is desirable to expand the number of T cells prior to electroporation, T cells can be stimulated to proliferate (e.g., by culture with PHA, PMA and/or OKT3, preferably in the presence of IL-2), followed by electroporation with RNA.

The inventors have discovered that the optimum conditions for electroporation of either resting or stimulated T cells is at a field strength of 100-150 Volts/mm gap width (e.g., 400-600V over a 4 mm gap) for 2-10 milliseconds (ms) using a square wave pulse. Preferably, the field strength is 110-140V/mm, more preferably 120-130V/mm, and most preferably about 125V/mm. For example, in an embodiment where 2 mm cuvettes are used, the most preferred voltage would be 250V. Preferably the voltage is applied for 3 to 7 ms, more preferably for 4 to 6 ms and most preferably for 5 ms. In preferred embodiments, the cells are electroporated in OptiMEM medium, or in a medium of similar conductivity at room temperature.

In one embodiment, the invention provides a method for transiently transfecting T cells, comprising electroporating T cells with RNA at a field strength of 100-150V/mm for 2-10 ms using a square wave pulse, wherein the T cells have not been stimulated in vitro by PHA or OKT3 prior to electroporation. Preferably the T cells are purified (e.g., purified CD8+ T cells, purified CD4+ T cells, or purified regulatory T cells) prior to electroporation. In another embodiment, the T cells are purified after electroporation.

T cells transiently transfected with TCR RNA are useful for the treatment of tumors, pathogen infection, autoimmune diseases, transplant rejection and GVHD. Thus in one embodiment, the invention provides the use of the T cell produced by the methods of the invention for the production of a medicament for immunotherapy. In another aspect, the invention includes a method for providing antigen-specific T cell effector function to a subject, comprising administering a T cell transiently transfected with RNA encoding a TCR specific for an antigen, wherein the T cell demonstrates effector function for cell presenting the antigen in complex with an MHC molecule. Preferably, the effector function is cytotoxicity, and the antigen is tumor-specific or pathogen-specific. In preferred embodiments, the T cell is autologous to the subject.

The T cell compositions of this invention can be co-administered with other therapeutic and cytotoxic agents, whether or not linked to them or administered in the same dosing. They can be coadministered simultaneously with such agents (e.g., in a single composition or separately) or can be administered before or after administration of such agents. Such agents can include immune stimulative cytokines like IL-2, chemotherapeutic drugs like cytostatica, antiviral-drugs, vaccines or any other kind of therapeutic agent abetting or amending the treatment.

### Methods for Making In Vitro Transcribed RNA

Certain embodiments of this invention require the preparation and use of IVT RNA. IVT RNA can be generated using any method known in the art. In preferred embodiments an expression cassette contains a promoter suitable for in vitro transcription, such as the T7 promoter or SP6 promoter. Preferably, the in vitro transcribed mRNA is optimized for stability and efficiency of translation. For example, mRNA stability and/or translational efficiency can be increased by including 3'UTRs and or 5'UTRs in the mRNA. Preferred examples of 3'UTRs include those from human β-actin (Qin and Gunning (1997) Journal of Biochemical and Biophysical Methods 36 pp. 63-72) and rotavirus gene 6 (Yang et. al., 2004 Archives of Virology 149:303-321). Preferred examples of 5'UTRs include the translational enhancers in the 5'UTRs of Hsp70 (Vivinus, et al., 2001 European Journal of Biochemistry 268:1908-1917), VEGF (Stein et al., 1998 Molecular and Cellular Biology 18:3112-3119), spleen necrosis virus RU5 (Roberts and Boris-Lawrie 2000 Journal of Virology 74:8111-8118), and tobacco etch virus (Gallie et al. (1995) Gene 165:233-238; Niepel and Gallie (1999) Journal of Virology 73:9080-9088. Gallie, Journal of Virology (2001) 75:12141-12152).

### Isolation of and Expansion of T Cells

T cells, including resting T cells and activated T cells, can be isolated from mammals by methods known to those of skill in the art. In non-limiting one method, Ficoll-Hypaque density gradient centrifugation is used to separate PBMC from red blood cells and neutrophils according to established procedures. Cells are washed with modified AIM-V (which consists of AIM-V (GIBCO) with 2 mM glutamine, 10 µg/ml gentamicin sulfate, 50 µg/ml streptomycin) supplemented with 1% fetal bovine serum (FBS). T cells are enriched by negative or positive selection with appropriate monoclonal antibodies coupled to columns or magnetic beads according to standard techniques. An aliquot of cells is analyzed for cell surface phenotype including CD4, CD8, CD3 and CD14. For the purpose of illustration only, cells are washed and resuspended at a concentration of about 5 X 10⁵ cells per ml of AIM-V modified as above and containing 5% FBS and 100 U/ml recombinant IL-2 (rIL-2) (supplemented AIM-V). Where the cells are isolated from and HIV⁺ patient, 25 nM CD4-PE40 (a recombinant protein consisting of the HIV-1-binding CD4 domain linked to the translocation and ADP-ribosylation domains of Pseudomonas aeruginosa exotoxin A), or other similar recombinant cytotoxic molecule which selectively hybridizes to HIV is added to the cell cultures for the remainder of the cell expansion to selectively remove HIV infected cells from the culture. CD4-PE40 has been shown to inhibit p24 production in HIV-infected cell cultures and to selectively kill HIV-1-infected cells. Preferred methods for isolating, culturing and expanding T cells are disclosed in the experimental section.

To stimulate proliferation, OKT3 monoclonal antibody (Ortho Diagnostics) can be added to a concentration of 10 ng/ml and the cells are plated in 24 well plates with 0.5 ml per well. The cells are cultured at a temperature of about 37°C in a humidified incubator with 5% CO₂ for 48 hours. Media is aspirated from the cells and 1 ml of vector-containing supernatant (described below) supplemented with 5 µl/ml of protamine sulfate, 100 U/ml rIL-2, 100 U/ml penicillin, 0.25 µg/ml amphotericin B/ml and an additional 100 µg/ml streptomycin (25 nM CD4-PE40 can be added). Methods for stimulating proliferation of T cells with PHA are disclosed in the Experimental section.

### Cell Isolation and Characterization

In another aspect, cell surface markers can be used to isolate or characterize the cells necessary to practice the method of this invention. For example, human stem cells typically express CD34 antigen while DCs express MHC molecules and costimulatory molecules (e.g., B7-1 and B7-2), a lack of markers specific for granulocytes, NK cells, B cells, and T cells. The expression of surface markers facilitates identification and purification of these cells. These methods of identification and isolation include FACS, column chromatography, panning with magnetic beads, western blots, radiography, electrophoresis, capillary electrophoresis, high performance liquid chromatography (HPLC), thin layer chromatography (TLC), hyperdiffusion chromatography, and the like, and various immunological methods such as fluid or gel precipitin reactions, immunodiffusion (single or double), immunoelectrophoresis, radioimmunoassays (RIAs), enzyme-linked immunosorbent assays (ELISAs), immunofluorescent assays, and the like. For a review of immunological and immunoassay procedures in general, see Stites and Terr (eds.) 1991 Basic and Clinical Immunology (7th ed.) and Paul supra. For a discussion of how to make antibodies to selected antigens see Harlow and Lane (1989) supra.

Cell isolation or immunoassays for detection of cells during cell purification can be performed in any of several configurations, e.g., those reviewed in Maggio (ed.) (1980) Enzyme Immunoassay CRC Press, Boca Raton, Fla.; Tijan (1985) "Practice and Theory of Enzyme Immunoassays," Laboratory Techniques in Biochemistry and Molecular Biology, Elsevier Science Publishers B.V., Amsterdam; Harlow and Lane, supra; Chan (ed.) (1987) Immunoassay: A Practical Guide Academic Press, Orlando, Fla.; Price and Newman (eds.) (1991) Principles and Practice of Immunoassays Stockton Press, NY; and Ngo (ed.) (1988) Non-isotopic Immunoassays Plenum Press, NY.

Cells can be isolated and characterized by flow cytometry methods such a FACS analysis. A wide variety of flow-cytometry methods are known. For a general overview of fluorescence activated flow cytometry see, for example, Abbas et al. (1991) Cellular and Molecular immunology W.B. Saunders Company, particularly chapter 3, and Kuby (1992) Immunology W.H. Freeman and Company, particularly chapter 6. FACS machines are available, e.g., from Becton Dickinson.

Labeling agents which can be used to label cell antigen include, but are not limited to monoclonal antibodies, polyclonal antibodies, proteins, or other polymers such as affinity matrices, carbohydrates or lipids. Detection proceeds by any known method, such as immunoblotting, western blot analysis, tracking of radioactive or bioluminescent markers, capillary electrophoresis, or other methods which track a molecule based upon size, charge or affinity.

### Therapeutic Uses

Transiently transfected CD8+ T cells can be introduced into a mammal where they are cytotoxic against target cells bearing antigenic peptides corresponding to those the T cells are manipulated to recognize with the introduced TCR on class I MHC molecules. Transfection of CD8+ T cells with RNA encoding an MHC class II restricted TCR allows for antigen-specific recognition of antigen:MHC class II complexes. Similarly, CD4+ helper T-cells recognize antigenic peptides in the context of MHC class II, but can also recognize peptide:MHC class I complexes when transfected with RNA encoding an MHC class I restricted TCR. Helper T-cells also stimulate an immune response against a target cell. The target cells are typically cancer cells, or pathogen infected cells.

The T cells can be isolated from the mammal into which the activated T cells are to be administered. Alternatively, the cells can be allogeneic provided from a donor or stored in a cell bank (e.g., a blood bank).

T cells produced by the methods of this invention can be administered directly to the subject to produce T cells active against a selected antigen. Administration can be by methods known in the art to successfully deliver a cell into ultimate contact with the most appropriate tissue(s). The cells are administered in any suitable manner, often with pharmaceutically acceptable carriers. Suitable methods of administering cells in the context of the present invention to a subject are available, and, although more than one route can be used to administer a particular cell composition, a particular route can often provide a more immediate and more effective reaction than another route. Preferred routes of administration include, but are not limited to intradermal, intravenous administration, lymph node administration and intratumoral administration. In one embodiment, the T cells are cotransfected with RNA encoding chemokine receptors or other homing molecules which guide the cells to sites in need of immunotherapeutic treatment, such as the site of metastases (e.g, intestine, liver, lungs, etc.), affected tissues in autoimmune diseases, etc. (For reviews of chemokine receptors and homing molecules, see: Salmi et al. Immunol Rev. 2005 206:100-13; Kim, Curr Opin Hematol. 2005 Jul;12(4):298-304; Kucia et al. Stem Cells. 2005 Aug;23(7):879-94; Ebert et al. Mol Immunol. 2005 May;42(7):799-809; Cambi et al. Cell Microbiol. 2005 Apr;7(4):481-8; Uhlig et al. Novartis Found Symp. 2004;263:179-88; discussion 188-92, 211-8; Kim et al. Curr Drug Targets Immune Endocr Metabol Disord. 2004 Dec;4(4):343-61: Zocchi et al. Leuk Lymphoma. 2004 Nov;45(11):2205-13; Sackstein J Investig Dermatol Symp Proc. 2004 Sep;9(3):215-23; Morris et al. Curr Mol Med. 2004 Jun;4(4):431-8; Marhaba et al J Mol Histol. 2004 Mar;35(3):211-31; Campbell et al. Semin Immunol. 2003 Oct;15(5):277-86; Cyster et al. Immunol Rev. 2003 Aug;194:48-60; Ley Trends Mol Med. 2003 Jun;9(6):263-8; Ono et al. J Allergy Clin Immunol. 2003 Jun;111(6):1185-99; the contents of which are incorporated by reference.

Pharmaceutically acceptable carriers are determined in part by the particular composition being administered, as well as by the particular method used to administer the composition. Accordingly, there is a wide variety of suitable formulations of pharmaceutical compositions of the present invention. Most typically, quality controls (microbiology, clonogenic assays, viability tests), are performed and the cells are reinfused back to the subject, preceded by the administration of diphenhydramine and hydrocortisone. See, for example, Korbling et al. (1986) Blood 67:529-532 and Haas et al. (1990) Exp. Hematol. 18:94-98.

Formulations suitable for parenteral administration, such as, for example, by intratumoral, intraarticular (in the joints), intravenous, intramuscular, intradermal, intraperitoneal, intranodal and subcutaneous routes, and carriers include aqueous isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. Intradermal and intravenous administration are the preferred method of administration for T cells of the invention.

The dose of cells (e.g., activated T cells, or dendritic cells) administered to a subject is in an effective amount, effective to achieve the desired beneficial therapeutic response in the subject over time, or to inhibit growth of cancer cells, or to inhibit infection.

For the purpose of illustration only, the method can be practiced by obtaining and saving blood samples from the subject prior to infusion for subsequent analysis and comparison. Generally at least about 10⁴ to 10⁶ and typically, between 1 X 10⁸ and 1 X 10¹⁰ cells are infused intravenously or intraperitoneally into a 70 kg patient over roughly 60-120 minutes. In one aspect, administration is by intratumoral injection. Vital signs and oxygen saturation by pulse oximetry are closely monitored. Blood samples are obtained 5 minutes and 1 hour following infusion and saved for analysis. Cell re-infusions are repeated roughly every month for a total of 10-12 treatments in a one year period. After the first treatment, infusions can be performed on an outpatient basis at the discretion of the clinician. If the re-infusion is given as an outpatient, the participant is monitored for at least 4 hours following the therapy.

For administration, cells of the present invention can be administered at a rate determined by the effective dose, the LD-50 (or other measure of toxicity) of the cell type, and the side-effects of the cell type at various concentrations, as applied to the mass and overall health of the subject. Administration can be accomplished via single or divided doses. The cells of this invention can supplement other treatments for a condition by known conventional therapy, including cytotoxic agents, nucleotide analogues and biologic response modifiers. Similarly, biological response modifiers are optionally added for treatment by the activated T cells of the invention. For example, the cells are optionally administered with an adjuvant, or cytokine such as GM-CSF, IL-12 or IL-2.

### Methods to Assess Immunogenicity

The immunogenicity of the T cells produced by the methods of the invention can be determined by well known methodologies including, but not limited to the following:

⁵¹Cr-release lysis assay. Lysis of peptide-pulsed ⁵¹Cr-labeled targets by antigen-specific T cells can be compared. "More active" compositions will show greater lysis of targets as a function of time. The kinetics of lysis as well as overall target lysis at a fixed timepoint (e.g., 4 hours) may be used to evaluate performance. In addition, the antigen density on the target cells that is required for killing indicates the T cell's affinity. Ware et al. (1983) 3. Immunol. 131:1312.

Cytokine-release assay. Analysis of the types and quantities of cytokines secreted by T cells upon contacting modified APCs can be a measure of functional activity. Methods for measuring cytokines include ELISA or ELISPOT assays to determine the rate and total amount of cytokine production. Fujihashi et al. (1993) J. Immunol. Meth. 160:181; Tanquay and Killion (1994) Lymphokine Cytokine Res. 13:259.

Proliferation Assays. T cells will proliferate in response to reactive compositions. Proliferation can be monitored quantitatively by measuring, for example, ³H-thymidine uptake. Caruso et al. (1997) Cytometry 27:71.

Transgenic animal models. Immunogenicity can be assessed *in vivo* by vaccinating HLA transgenic mice with the compositions of the invention and determining the nature and magnitude of the induced immune response. Alternatively, the hu-PBL-SCID mouse model allows reconstitution of a human immune system in a mouse by adoptive transfer of human PBL. These animals may be vaccinated with the compositions and analyzed for immune response as previously mentioned in Shirai et al. (1995) J. Immunol. 154:2733; Mosier et al. (1993) Proc. Natl. Acad. Sci. USA 90:2443.

Primate models. A non-human primate (chimpanzee) model system can be utilized to monitor in *vivo* immunogenicities of HLA-restricted ligands. It has been demonstrated that chimpanzees share overlapping MHC-ligand specificities with human MHC molecules thus allowing one to test HLA-restricted ligands for relative *in vivo* immunogenicity. Bertoni *et al.* (1998) Immunol. 161:4447.

Monitoring TCR Signal Transduction Events. Several intracellular signal transduction events (e.g., phosphorylation) are associated with successful TCR engagement by MHC-ligand complexes. The qualitative and quantitative analysis of these events have been correlated with the relative abilities of compositions to activate effector cells through TCR engagement. Salazar et al. (2000) Tnt. J. Cancer 85:829; Isakov et al. (1995) J. Exp. Med. 181:375).

In accordance with the above description, the following examples are intended to illustrate, but not limit, the various aspects of this invention.

### EXPERIMENTAL

### Materials & Methods

### Cells and reagents

PBMC were prepared from whole blood of healthy donors (obtained following informed consent and approved by the institutional review board) by density centrifugation using Lymphoprep (Axis-Shield, Oslo, Norway). For the generation of dendritic cells (DC) and nonadherent fraction (NAF), PBMC were resuspended in autologous medium that consisted of RPMI 1640 (Cambrex) containing 1% heat-inactivated autologous plasma, 2 mM L-glutamine (BioWhittaker), 20 mg/L gentamicin (Sigma-Aldrich) and were transferred to tissue culture dishes (BD Falcon), at 30x10⁶ cells/dish. Cells were incubated for 1-2 h at 37°C to allow for adherence, the nonadherent fraction was removed and mature DC were generated from the adherent cells as described previously [22]. Matured DC were harvested and electroporated with gp100 RNA as described previously [22]. CD8⁺ T cells were isolated using anti-CD8 MACS beads (Miltenyi, Bergisch Gladbach, Germany) according to the manufacturer's instructions. T cells were cultured in MLPC medium consisting of RPMI 1640, 10% human serum, 2 mM L-glutamine, 20mg/L gentamicin, 10 mM HEPES, 1 mM sodium pyruvate (Sigma-Aldrich), 1% MEM nonessential amino acids (100x), supplemented with 20 U/ml IL-7. Twenty IU/ml IL-2 and 20 U/ml IL-7 were added on days 2 and 4. For the generation of phytohemaglutinin (PHA)-stimulated T-cell cultures, 2x10⁶/ml NAF were cultured in T25 culture flasks in AIM-V medium (Invitrogen) supplemented with 10% human serum, 1 µg/ml PHA (Sigma), 20 U/ml IL-7 and 20 IU/ml IL-2. IL-2 and IL-7 were added every two days.

The melanoma cell lines SK-MEL526 (HLA-A2⁺/gp100⁺), Colo 829 (HLA-A1⁺/A2⁻/gp100⁺) and NEMA (HLA-A2⁺/gp100⁻), and the T2 cells (a TAP-deficient TxB cell hybrid T2-A1 (HLA-A1⁺/A2⁺; ATCC# CRL-1992) were cultured in R10 medium consisting of RPMI 1640, 2 mM L-Glutamine, Penicillin-Streptomycin, 10% fetal calf serum, 2 mM HEPES and 2-βME. Gp100 expression of the melanoma cell lines was confirmed by intracellular staining with the mouse anti-gp100 mAb HMB45 (DAKO, Glostrup, Denmark) and donkey anti-mouse-PE (RDI, Concord, MA, USA). Specifically, cells were permeabilized with Cytofix/Cytoperm solution (BD Biosciences, Heidelberg, Germany), and stained with primary and secondary Abs according to the manufacturer's instructions.

TCR-transfected T cells were analyzed for TCR expression by flow cytometry using PE-conjugated anti-TCRVβ14 mAb (i.e., recognizing the gp100/A2-specific TCR), or PE-labeled gp100/HLA-A2 tetramer (Proimmune, Oxford, UK).

Peptides used in this study were: the HLA-A2-binding gp100₂₀₉₋₂₁₇ analogue IMDQVPFSV, and gp100₂₈₀₋₂₈₈ YLEPGPVTA.

### Cloning of TCR genes

The cloning of the gp100-specific 296 TCR genes into the retroviral pBullet vector was described before [24]. The coding sequences of the TCR 296 α chain was re-cloned from the retroviral pBullet vector (kindly provided by Dr. R. Debets, ErasmusMC, Rotterdam) into the pGEM4Z-5'UTR-sig-MAGE-A3-DC.LAMP-3'UTR vector [4] (kindly provided by Dr. K. Thielemans, VUB, Brussels), by digesting both with *Nco*I and *Xho*I. The pGEM4Z-5'UTR-sig-MAGE-A3-DC.LAMP-3'UTR vector contains the 5' and 3' untranslated regions of the *Xenopus laevis* β-globin gene and a poly-A tail. At the 3' end of the poly-A tail, unique *Not*I and *Spe*I sites are present to allow linearization of the plasmids before in vitro transcription. A bacteriophage T7 promoter allows the in vitro generation of mRNA. The coding sequences of the TCR β 296 chain was first amplified by PCR using the following primers:
-TCRB296*Bam*HI
5'-CTC TGG ATC C _{*Bam*HI} AT GGG CCC CCA GCT CCT TGG CTA TG-3'
-HCB
5'CTC TCT CGA G _{*Xho*I} GG ATC GCT AGC CTC TGG AAT CCT TTC TC-3'.
The PCR product was digested with *Bam*HI and *Xho*I and cloned into the pGEM4Z-5'UTR-sig-MAGE-A3-DC.LAMP-3'UTR vector which was digested with *Bgl*II and *Xho*I.

### In vitro transcription of TCR RNA

The pGEM4Z-gp100-64A vector was generated by cloning the gp100 gene into the pGEM4Z-64A vector [1] and deleting the *Sph* I site (alternative starting codon) by site-directed mutagenesis. For *in vitro* transcriptions, a pGEM4Z-enhanced GFP vector, pGEM4Z-TCRα296, pGEM4Z-TCRβ296, and pGEM4Z-gp100 were linearized with *Spe*I enzyme, purified with phenol/chloroform extraction and ethanol precipitation, and used as DNA templates [14]. The *in vitro* transcription was performed with T7 RNA polymerase (mMESSAGE mMACHINE T7 Ultra kit; Ambion) according to the manufacturer's instructions. The in vitro transcribed (IVT) RNA was recovered after DNaseI (Ambion) digestion on RNeasy columns (Qiagen) according to the manufacturer's instructions. RNA quality was verified by agarose gel electrophoresis, RNA concentration was measured spectrophotometrically, and RNA was stored at -80°C in small aliquots.

### RNA electroporation of T lymphocytes

CD8+ T cells were harvested from dishes and washed once with pure RPMI 1640 and once with OptiMEM without phenol red (Invitrogen Life Technologies) (all at room temperature). The cells were resuspended in OptiMEM at a concentration of 8 x 10⁷/ml. IVT RNA was transferred to a 4-mm cuvette (Peqlab)(150 µg/ml final concentration). A volume of 100-600 µl of cell suspension was added and incubated for 3 min before being pulsed in a Genepulser Xcell (Bio-Rad). Pulse conditions were square-wave pulse, 500 V, 5 ms. Immediately after electroporation, the cells were transferred to MLPC medium supplemented with the previously indicated concentrations of IL-7, and IL-2 where indicated.

### Cryopreservation of T cells

Cryopreservation was performed as follows: cells were taken up in 20% HSA (Pharmacia & Upjohn) at a concentration of 20-50 x 10⁶ cells/ml, and stored for 10 min on ice. An equal volume of cryopreservation medium, i.e., 55% HSA (20%), 20% DMSO (Sigma-Aldrich), and 25% glucose (Glucosteril 40; Fresenius), was added to the cell suspension. Cells were then frozen at -1°C/min in a cryofreezing container (Nalgene) to -80°C. Thawing was performed by holding cryotubes in a 37°C water-bath until detachment of the cells was visible. Cells were then poured into 10 ml of RPMI 1640, washed, and added to a cell culture dish containing prewarmed MLPC medium with 20 U IL-7/ml. Cells were rested for 0,5 h in a 37°C incubator before additional experiments.

### Flowcytometry of TCR-transfected T lymphocytes

For surface stainings with anti-TCRVβ mAb, T cells were washed and thereafter suspended at 1 x 10⁵ cells in 100 µl of cold FACS solution (Dulbecco's PBS; Bio Whittaker) containing 0.1% sodium azide (Sigma-Aldrich) and 0.2% HSA (Octapharma) and incubated with mAb for 30 min. Cells were then washed and resuspended in 100 µl of cold FACS solution. Stained cells were analyzed for two-color immunofluorescence with a FACStar cell analyzer (BD Biosciences). Cell debris was eliminated from the analysis using a gate on forward and side light scatter. A minimum of 10⁴ cells was analyzed for each sample. Results were analyzed using CellQuest software (BD Biosciences).

For tetramer staining, a total of 10⁶ T cells were resuspended in 90 µl of RPMI 1640 supplemented with 5% pooled-serum, 10 mM HEPES, 1 mM sodium pyruvate, 1% MEM nonessential amino acids (100x), 2 mM L-glutamine, and 20 mg/L gentamicin. Five hundred nanograms oftetramer was added. T cell phenotype was analyzed by flow cytometry using anti-CCR7 FITC and anti-CD45RA ECD (phycoerythrin-Texas Red). Cells were incubated for 20 min at 37°C, 5% CO₂, and then cooled to 4°C. The cells were washed, and analyzed on a CYTOMICS FC500 from Beckman Coulter.

### Induction and determination of IFN-γ production by TCR-transfected T lymphocytes

T cells electroporated with TCR RNA were cocultivated with irradiated (0,005J/cm²) T2 cells which were loaded with an irrelevant peptide (gp100/A2₂₀₉₋₂₁₇ analogue IMDQVPFSV) or the peptides recognized by the TCR (gp100/A2₂₈₀₋₂₈₈ YLEPGPVTA)(both at 10µM) for 1 h at 37°C. Fifteen thousand T cells were cocultivated with 15,000 T2 cells in a volume of 100 µl of RPMI 1640 (Cambrex) supplemented with 10% pooled-plasma (heat-inactivated and sterile-filtered plasma from healthy donors), 10 mM HEPES (Sigma-Aldrich), 1 mM sodium pyruvate (Sigma-Aldrich), 1% MEMnonessential amino acids 100x (Sigma-Aldrich), 2 mM L-glutamine (Cambrex), 20 mg/L gentamicin (Sigma-Aldrich), and 20 IU/ml IL-2. Supernatants were harvested after 16 h and IFN-γ production was determined using a commercially available ELISA kit according to the manufacturer's protocol (DPC Biermann).

### Cytotoxicity assay

Cytotoxicity was tested in standard 4-6 h ⁵¹Cr release assays. In short, T2 target cells were labeled with 100 µCi of Na₂⁵¹ CrO₄/10⁶ cells for 1 h at 37°C/5% CO₂, washed, loaded with peptides for 1 h at 37°C/5% CO₂, and washed again before cocultivation with effector T cells. Peptides were loaded at a concentration of 10 µM, or as indicated. As alternative target cells gp100 RNA-transfected, HLA-A2⁺DC, or the melanoma cell lines SK-MEL562, Colo829 and NEMA were used. Target cells were added to 96-well plates at 1000 cells/well. Effector cells, i.e., TCR-transfected T cells, were added at an E:T ratio of 60:1, 20:1, 7:1, and 3:1. Percentage cytolysis, i.e., ⁵¹Cr release, was calculated as follows: [(measured release - background release)]/[(maximum release - background release)] x 100%.

### Results

### T cells are efficiently transfected with RNA

Optimized and reproducible transfection of CD8+ T cells was achieved by a step-wise development of the electroporation protocol, using EGFP RNA as a model. CD8⁺ T cells of several donors were transfected, according to this optimized protocol, with RNA coding for EGFP or the α and β chains of the TCR originating from CTL clone specific for HLA-A2-presented gp100 peptide (296 CTL clone). Expression levels of EGFP and TCR chains was determined by flow cytometry. Approximately 93% of the electroporated T cells expressed EGFP (Fig. 1) (MFI=135) 4h after electroporation, pointing to a very high transfection efficiency of T cells.

A low, but significant TCR β chain expression of the gp100-specific TCR was detected with an anti-TCRVβ14 mAb on the cell membrane 4h (p=0,0027) and 24h (p=0,0025) after electroporation (Fig. 1b). However, no binding of HLA-A2/gp100₂₈₀₋₂₈₈ tetramers was observed when TCR-transfected T cells were stained (data not shown), even though transfection efficiency of EGFP RNA under the same conditions was >90% (Fig. 1a and b). Nonetheless, the non-activated TCR-RNA-electroporated CD8⁺ T cells were functional in both IFNγ release and cytotoxicity assays. Undetectable tetramer binding may be due to low receptor density on the cell membrane of our TCR-RNA-electroporated T cells, since tetramers need to be bound by several TCRs at the same time to adhere firmly to the T cell membrane. If the TCR molecules are too far apart, only monovalent binding of the tetramer is possible, which results into low avidity [17]. Nevertheless, the TCR expression in transiently transfected T cells was sufficient to trigger them to lyse targets and produce IFNγ.

Mock-transfected T cells did not show any expression of EGFP, and only endogenous expression of the TCR β chain (Fig. 1a and b). In addition, the T cell phenotype of non-electroporated, mock-electroporated, and TCR RNA-electroporated T cells was determined by FACS staining for CCR7 and CD45RA. As shown in Fig. 1c, there was no influence of electroporation on the phenotype of bulk electroporated T cells compared to non-electroporated T cells.

### Antigen-positive target cells specifically stimulate TCR-transfected T cells to produce IFNγ

Although the measured TCR expression was low, CD8⁺ T cells transfected with TCR RNA were tested for their cytokine production capacity in response to target cells loaded with gp100₂₈₀₋₂₈₈ peptide at 4h, 24h and 48h after electroporation. As shown in figure 2, only T cells transfected with RNA coding for the gp100/A2 specific TCR responded to T2 cells loaded with the gp100₂₈₀₋₂₈₈ peptide with IFNγ production, while T cells electroporated with EGFP RNA were not able to produce IFNγ. Furthermore, T2 cells loaded with a control peptide (i.e. gp100₂₀₉₋₂₁₇ analogue) were not able to induce IFNγ production by RNA-transfected T cells (Fig. 2). Even at 48h after electroporation, there was a clear specific IFNγ production by T cells transfected with RNA coding for the gp100/A2-specific TCR (Fig. 2). Next, we tested whether TCR-transfected T cells could by cryopreserved without loss of IFNγ production capacity. Figure 3a shows that, the T cells frozen 4h after transfection with RNA coding for the gp100/A2-specific TCR, still produced IFN γ when they were stimulated with gp100₂₈₀₋₂₈₈-peptide-loaded T2 cells directly after thawing. Moreover, IFNγ production by TCR-transfected T cells was determined after incubation with RNA-electroporated DC. Only DC electroporated with gp 100 RNA, but not mock-electroporated DC electroporated, were able to stimulate TCR-transfected T cells to produce IFNγ (Fig. 3b).

### Peptide-loaded targets and melanoma cells are specifically lysed by TCR-transfected T cells

CD8⁺ T cells transfected with TCR RNA were tested for their cytolytic capacity on target cells loaded with gp100₂₈₀₋₂₈₈ peptide at 24h, 48h and 72h after electroporation. As shown in figure 4a (representative figure at the 48h time-point), only T2 cells loaded with the gp100₂₈₀₋₂₈₈ peptide were lysed by TCR RNA-transfected T cells. T2 cells loaded with a control peptide were not lysed, and T cells transfected with EGFP RNA did not lyse any of the targets (Fig. 4a). A time-course of cytolysis by RNA-transfected T cells was performed (Fig. 4b). As measured in cytotoxicity time-courses, the T cells were still very lytic 3 days after electroporation in all measured target:effector ratios. A specific lysis of T2 cells loaded with the gp100₂₈₀₋₂₈₈ peptide at an target:effector ratio of 1:20 was observed at all time-points (Fig. 4b). At all other measured target:effector ratios a specific lysis was seen (data not shown). Furthermore, at the highest target:effector ratio (i.e. 1:60) a specific lysis was still observed one week after electroporation of the T cells (data not shown), pointing to the longevity of specific lysis of TCR RNA-electroporated T cells. The fact that the specific lysis of target cells is stable over several days is of eminent importance for the use of TCR-transfected CD8⁺ T cells in immunotherapy of cancer. Similar results in IFNγ secretion and cytotoxicity assays were achieved with PHA/IL-2/IL-7-stimulated T cells, which were magnetically selected for CD8 after three days of stimulation and then RNA electroporated. PHA/IL-2/IL-7 stimulation results in the proliferation T cells and is useful for the generation of large numbers of TCR RNA-electroporated T cells for immunotherapy of cancer.

Moreover, the cytolytic capacity of the TCR RNA-electroporated T cells was preserved after cryopreservation, and T cells that were PHA-stimulated and expanded before electroporation, were able to produce IFNγ in response to peptide-loaded target cells, and also lysed these cells (data not shown). Stability of the cytolytic capacity of the TCR RNA-electroporated T cells after cryopreservation makes it possible to generate multiple doses of the T vaccine in a single process (and from a single leukopheresis) for repetitive administration to a patient.

Importantly, TCR RNA-electroporated T cells (4h after electroporation) were also able to specifically recognize and lyse tumor cells that are gp100⁺ *and* HLA-A2⁺ (Fig. 5, SK-MEL526). Tumor cell lines that were gp100- but HLA-A2⁺ (NEMA), or gp100⁺ but HLA-A2⁻ (Colo829) were not recognized by the TCR RNA-electroporated T cells (Fig. 5). EGFP RNA-electroporated T cells did not lyse any of the target cells (Fig. 5).

### The cytolytic efficiency of TCR RNA- transfected T cells is similar to that of retrovirally transduced T cells and approximates that of the parental CTL clone

From previous published work, we know that the cytolytic efficiency of the parental 296 CTL clone was preserved following retroviral transduction of TCR genes into T cells [24]. To test the avidity of the TCR RNA-transfected T cells, we performed a cytotoxicity assay (24h after electroporation) in which we titrated the gp100₂₈₀₋₂₈₈ peptide on T2 target cells (Fig. 6). The peptide concentration corresponding to 50% of the maximum lysis (i.e. ED₅₀) of the TCR RNA-transfected T cells ranged from 300-1000 pM in three independent experiments (Fig. 6). This is in the same range as the cytolytic efficiency of the retrovirally transduced T cells (ED₅₀=300 pM). Both approximate the cytolytic efficiency of the parental 296 CTL clone, which has an ED₅₀ of 50 pM [24]. EGFP RNA-transfected T cells did not lyse T2 cells loaded with the gp100₂₈₀₋₂₈₈ peptide (10µM) (Fig. 6), and T2 cells loaded without peptide were not lysed by the TCR RNA-transfected T cells (data not shown). Taken together, the TCR RNA-transfected T cells lyse targets with high avidity, similar to retrovirally transduced T cells. The high cytolytic efficiency makes TCR RNA transfected CD8⁺ T cells a practicable alternative to adoptive transfer of expanded tumor-specific CTL (TIL) clones.

The results herein demonstrate that CD8⁺ T cells electroporated with RNA coding for the TCR α and β chain originating from an HLA-A2/gp100₂₈₀₋₂₈₈-specific CTL clone gain lytic effector function. Functionality of these TCR-transfected T cells was tested along several lines: 1) specific IFNγ production 4h, 24h, and 48h after electroporation in response to stimulation with peptide-loaded target cells (Fig. 2), 2) specific IFNγ production after cryopreservation (i.e. 0h, 24h, and 48h after thawing) in response to stimulation with peptide-loaded target cells (Fig. 3a), 3) specific IFNγ production in response to stimulation with gp100-RNA-electroporated dendritic cells (Fig. 3b), 4) specific cytolysis of peptide-loaded target cells 24h, 48h, 72h (Fig. 4), and one week (data not shown) after electroporation, 5) specific cytolysis of a HLA-A2⁺/gp100⁺ melanoma cell line (Fig. 5), and 6) cytolytic efficiency using peptide-loaded target cells (Fig. 6). This is the first description of the transfer of cytolytic function by TCR-coding RNA transfection of T cells.

### Electroportion of CD4+ T cells with RNA encoding a TCR acquire the specificity of that TCR

The purpose of the described experiments was to show that CD4 T cells electroporated with RNA coding for a TCR acquire the specificity of that TCR. CD4+ T cells were electroporated with RNA encoding one of two different TCRs. The first TCR is specific for an MHC Class II DP4 restricted peptide derived from the cancer-testis antigen Mage3 (M3-DP4), and was cloned from a CD4+ T cell clone. The second TCR is specific for a MHC Class I A2 restricted peptide derived from the melanoma antigen gp100 (gp100-A2), and was cloned from a CD8+ T cell clone, as described above. For the first TCR, autologous mature DC were used as target, and for the second TCR, T2 cells. Both targets were loaded with the corresponding peptide. The M3-DP4 TCR transfected T cells specifically produced IFN-γ, TNF, and IL-2, some IL-4 and little IL-10. The gp100 TCR transfected T cells produced high amounts of IFN-γ, TNF, and IL-2, but also some IL-4 and IL-10. This data, for the first time, show that electroporation of purified CD4⁺ T cells with RNA, coding for a TCR, generates T cells specifically recognizing the TCR's target. This will allow manipulating T cells help efficiently and easily in therapy and research.

As readout the Cytometric Bead Array (CBA) from BD was chosen, which allows the measure 6 different cytokines at once (Fig. 7). We chose IL-2, IL-4, IL-6 IL-10, TNF, and IFN-gamma. Two different TCR were used in the experiments: 1 a TCR specific for a MHC Class II DP4 restricted peptide, derived from the cancer-testisantigen Mage3 (M3-DP4), that was cloned from a CD4⁺ T cell clone. 2^{nd} the TCR has a very affinity specific for a MHC Class I A2 restricted peptide derived from the melanoma antigen gp100 (gp100-A2), that was generated from a CD8 T cell clone and that we have already successfully used in CD8 cells.

In addition a 3rd batch of T cells from the same preparation was transfected with EGFP, to determine transfection efficiency, and as negative control for the cytokine release. As target for the M3-DP4 TCR electroporated T cells autologous mature (cocktail) DC were used, while T2 cells were used for gp100-A2. Effectors and targets were cocultured, and after 20 h and 44 h samples from the supernatant were taken, and tested for cytokine content. At the 20 h time point, no standard curve was prepared, since this was our first CD4+ experiment that worked, and we only wanted to see if something happens. At the 44 h time point, a standard curve was prepared. However, some cytokines were produced so efficiently, that they were out of the range of that curve.

The transfection efficiency was tested by electroporating GFP-RNA, and FACS analysis 24h later (Fig. 8). The tranfection efficiency was 86%, and the mean fluorescence was 127. Judged by the FSC SSC of the cells, the viability was also very high with 95% in the life gate.

The CD4+ T cells electroporated with Mage3 DP4 specific TCR were cocultured with autologous mature DC that were loaded with the corresponding Mage3 DP4 peptide. As controls, the same DC, but without peptide were used. In addition, CD4+ T cells transfected with EGFP were used as negative control. Samples were taken after 20 h and after 44 h, and were analysed by a CBM. No standard curve was prepared for the 20 h time point, so the provided data are only semi-quantitative, as they are only the MFIs of the cytokine-binding beads. Nevertheless the data show a very clearly that the TCR-RNA electroporated CD4+ cells specifically recognize their target, but not the control target, and also the control effectors recognize nothing (Fig 9). The CD4+ cells produced large amounts of IFNy, TNF, and IL-2, but also some IL-4 (note that the scale is logarithmic). Of note is that the DC by them self secrete some IL-6. This is not an unspecific reaction of the T cells, since DC without T cells did the same (not shown).

After 44 h another sample was taken from the supernatant, and again analyzed by CBI. This time a standard curve was prepared, to get an idea of the absolute numbers. The supernatants were this time diluted 1:2. Since the IFNγ was above the highest standard value, the MFIs are also indicated (Fig 10). The data show that still specific reaction can be observed, however the numbers are not as nice as after 20 h. Probably some unspecific reaction occurred, leading to the loss of specific IL-2 release, and some of the cytokines may start to degrade again.

The CD4+ T cells electroporated with gp100 specific TCR were cocultured with T2 cells, a cell line that expresses HLA A2 but presents no or little endogenous peptide and that can be efficiently loaded with exogenous peptide. These cells were loaded with the gp100-A2 peptide. As controls, T2 cells, but without peptide, were used. In addition, CD4+ T cells transfected with EGFP were used as additional negative control. Samples were taken after 20 h and after 44 h, and were analyzed by a CBM. Again the data from 20 h time point are only semi-quantitative. Nevertheless the data show very clearly that the TCR-RNA electroporated CD4+ cells specifically recognize their target, but not the control target, and also the control effectors recognize nothing (Fig 11). The CD4+ cells produced even larger amounts of IFNγ, TNF, and IL-2, than with the M3-DP4 TCR, but also some IL-4 and IL-10 (note that the scale is logarithmic).

After 44 h another sample was taken from the supernatant, and again analyzed by CBI. This time a standard curve was prepared, to get an idea of the absolute numbers. The supernatants were also diluted 1:2. Since the IFNy and the IL-2 was above the highest standard value for the gp100-A2 TCR transfected CD4+ cells on their specific target, the MFIs are also indicated (Fig 12). The data show that still a strong and clean specific reaction can be observed, which does not differ substantially from the 20 h time point. No unspecific reaction occurred, probably because the T2 cells do not express costimulatory molecules.

So in aggregate these data show for the first time that purified CD4+ T cells can be RNA electroporated with high efficiency, and, more importantly, that HLA class I and II restricted TCRs can be functionally expressed in these cells using the method of RNA electroporation. This opens up new possibilities to provide or manipulate T cells help in immunotherapy but also in research and development.

### REFERENCES

1. Boczkowski D, Nair SK, Nam JH, Lyerly HK, Gilboa E (2000) Induction of tumor immunity and cytotoxic T lymphocyte responses using dendritic cells transfected with messenger RNA amplified from tumor cells. Cancer Res. 4:1028-34
2. Bolhuis RL, Gratama JW (1998) Genetic re-targeting of T lymphocyte specificity. Gene Ther. 9:1153-55
3. Bolhuis RL, Willemsen RA, Gratama JW (2000) Clinical applications of redirected cytotoxicity.Sitkovsky MV, Henkart PA (eds) cytotoxic cells. Lippincott, Williams & Wilkins, Philadelphia
4. Bonehill A, Heirman C, Tuyaerts S, Michiels A, Breckpot K, Brasseur F, Zhang Y, Van Der BP, Thielemans K (2004) Messenger RNA-electroporated dendritic cells presenting MAGE-A3 simultaneously in HLA class I and class II molecules. J.Immunol. 11:6649-57
5. Bonini C, Grez M, Traversari C, Ciceri F, Marktel S, Ferrari G, Dinauer M, Sadat M, Aiuti A, Deola S, Radrizzani M, Hagenbeek A, Apperley J, Ebeling S, Martens A, Kolb HJ, Weber M, Lotti F, Grande A, Weissinger E, Bueren JA, Lamana M, Falkenburg JH, Heemskerk MH, Austin T, Kornblau S, Marini F, Benati C, Magnani Z, Cazzaniga S, Toma S, Gallo-Stampino C, Introna M, Slavin S, Greenberg PD, Bregni M, Mavilio F, Bordignon C (2003) Safety of retroviral gene marking with a truncated NGF receptor. Nat.Med. 4:367-69
6. Buckley RH (2002) Gene therapy for SCID--a complication after remarkable progress. Lancet 9341:1185-86
7. Clay TM, Custer MC, Sachs J, Hwu P, Rosenberg SA, Nishimura MI (1999) Efficient transfer of a tumor antigen-reactive TCR to human peripheral blood lymphocytes confers anti-tumor reactivity. J.Immunol. 1:507-13
8. Debets R, Willemsen R, Bolhuis R (2002) Adoptive transfer of T-cell immunity: gene transfer with MHC-restricted receptors. Trends Immunol. 9:435-36
9. Dudley ME, Wunderlich JR, Robbins PF, Yang JC, Hwu P, Schwartzentruber DJ, Topalian SL, Sherry R, Restifo NP, Hubicki AM, Robinson MR, Raffeld M, Duray P, Seipp CA, Rogers-Freezer L, Morton KE, Mavroukakis SA, White DE, Rosenberg SA (2002) Cancer regression and autoimmunity in patients after clonal repopulation with antitumor lymphocytes. Science 5594:850-854
10. Economou JS, Belldegrun AS, Glaspy J, Toloza EM, Figlin R, Hobbs J, Meldon N, Kaboo R, Tso CL, Miller A, Lau R, McBride W, Moen RC (1996) In vivo trafficking of adoptively transferred interleukin-2 expanded tumor-infiltrating lymphocytes and peripheral blood lymphocytes. Results of a double gene marking trial. J.Clin.Invest 2:515-21
11. Figlin RA, Thompson JA, Bukowski RM, Vogelzang NJ, Novick AC, Lange P, Steinberg GD, Belldegrun AS (1999) Multicenter, randomized, phase III trial of CD8(+) tumor-infiltrating lymphocytes in combination with recombinant interleukin-2 in metastatic renal cell carcinoma. J.Clin.Oncol. 8:2521-29
12. Hacein-Bey-Abina S, Le Deist F, Carlier F, Bouneaud C, Hue C, De Villartay JP, Thrasher AJ, Wulffraat N, Sorensen R, Dupuis-Girod S, Fischer A, Davies EG, Kuis W, Leiva L, Cavazzana-Calvo M (2002) Sustained correction of X-linked severe combined immunodeficiency by ex vivo gene therapy. N.Eng1.J.Med. 16:1185-93
13. Hanson HL, Donermeyer DL, Ikeda H, White JM, Shankaran V, Old LJ, Shiku H, Schreiber RD, Allen PM (2000) Eradication of established tumors by CD8+ T cell adoptive immunotherapy. Immunity. 2:265-76
14. Heiser A, Dahm P, Yancey DR, Maurice MA, Boczkowski D, Nair SK, Gilboa E, Vieweg J (2000) Human dendritic cells transfected with RNA encoding prostate-specific antigen stimulate prostate-specific CTL responses in vitro. J.Immunol. 10:5508-14
15. Marshall E (2002) Clinical research. Gene therapy a suspect in leukemia-like disease. Science 5591:34-35
16. Morgan RA, Dudley ME, Yu YY, Zheng Z, Robbins PF, Theoret MR, Wunderlich JR, Hughes MS, Restifo NP, Rosenberg SA (2003) High efficiency TCR gene transfer into primary human lymphocytes affords avid recognition of melanoma tumor antigen glycoprotein 100 and does not alter the recognition of autologous melanoma antigens. J.Immunol. 6:3287-95
17. Ogg GS, McMichael AJ (1998) HLA-peptide tetrameric complexes. Curr.Opin.Immunol. 4:393-96
18. Parmiani G, Castelli C, Rivoltini L, Casati C, Tully GA, Novellino L, Patuzzo A, Tosi D, Anichini A, Santinami M (2003) Immunotherapy of melanoma. Semin.Cancer Biol. 6:391-400
19. Restifo NP, Wunderlich JR (1996) Principles of tumor immunity: biology of cellular immune responses.DeVita VT, Hellman S, Rosenberg SA (eds) Biologic Therapy of Cancer. Lippincott Co, Philadelphia
20. Rosenberg SA (1999) A new era of cancer immunotherapy: converting theory to performance. CA Cancer J.Clin. 2:70-3, 65
21. Roszkowski JJ, Lyons GE, Kast WM, Yee C, Van Besien K, Nishimura MI (2005) Simultaneous generation of CD8+ and CD4+ melanoma-reactive T cells by retroviral-mediated transfer of a single T-cell receptor. Cancer Res. 4:1570-1576
22. Schaft N, Dorrie J, Thumann P, Beck VE, Muller I, Schultz ES, Kampgen E, Dieckmann D, Schuler G (2005) Generation of an optimized polyvalent monocyte-derived dendritic cell vaccine by transfecting defined RNAs after rather than before maturation. J.Immunol. 5:3087-97
23. Schaft N, Lankiewicz B, Gratama JW, Bolhuis RL, Debets R (2003) Flexible and sensitive method to functionally validate tumor-specific receptors via activation of NFAT. J.Immunol.Methods 1-2:13-24
24. Schaft N, Willemsen RA, de Vries J, Lankiewicz B, Essers BW, Gratama JW, Figdor CG, Bolhuis RL, Debets R, Adema GJ (2003) Peptide Fine Specificity of Anti-Glycoprotein 100 CTL Is Preserved Following Transfer of Engineered TCRalphabeta Genes Into Primary Human T Lymphocytes. J.Immunol. 4:2186-94
25. Scheel B, Teufel R, Probst J, Carralot JP, Geginat J, Radsak M, Jarrossay D, Wagner H, Jung G, Rammensee HG, Hoerr I, Pascolo S (2005) Toll-like receptor-dependent activation of several human blood cell types by protamine-condensed mRNA. Eur. J. Immunol. 35:1557-66
26. Smits E, Ponsaerts P, Lenjou M, Nijs G, Van Bockstaele DR, Berneman ZN, Van Tendeloo VF (2004) RNA-based gene transfer for adult stem cells and T cells. Leukemia 11:1898-902
27. Stanislawski T, Voss RH, Lotz C, Sadovnikova E, Willemsen RA, Kuball J, Ruppert T, Bolhuis RL, Melief CJ, Huber C, Stauss HJ, Theobald M (2001) Circumventing tolerance to a human MDM2-derived tumor antigen by TCR gene transfer. Nat.Immunol. 10:962-70
28. Theobald M, Biggs J, Hernandez J, Lustgarten J, Labadie C, Sherman LA (1997) Tolerance to p53 by A2.1-restricted cytotoxic T lymphocytes. J.Exp.Med. 5:833-41
29. Willemsen RA, Weijtens ME, Ronteltap C, Eshhar Z, Gratama JW, Chames P, Bolhuis RL (2000) Grafting primary human T lymphocytes with cancer-specific chimeric single chain and two chain TCR. Gene Ther. 16:1369-77
30. Xue S, Gillmore R, Downs A, Tsallios A, Holler A, Gao L, Wong V, Morris E, Stauss HJ (2005) Exploiting T cell receptor genes for cancer immunotherapy. Clin.Exp.Immunol. 2:167-72
31. Yee C, Thompson JA, Byrd D, Riddell SR, Roche P, Celis E, Greenberg PD (2002) Adoptive T cell therapy using antigen-specific CD8+ T cell clones for the treatment of patients with metastatic melanoma: in vivo persistence, migration, and antitumor effect of transferred T cells. Proc.Natl.Acad.Sci.U.S.A 25:16168-73
32. Zhang T, He X, Tsang TC, Harris DT (2004) Transgenic TCR expression: comparison of single chain with full-length receptor constructs for T-cell function. Cancer Gene Ther. 7:487-96

### Annex to the application documents - subsequently filed sequences listing

SEQUENCE LISTING
TCR alpha chain
ATGGCATCCATTCGAGCTGTATTTATATTCCTGTGGCTGCAGCTGGACTTGGTGAATGGAGA
GAATGTGGAGCAGCATCCTTCAACCCTGAGTGTCCAGGAGGGAGACAGCGCTGTTATCAAG
TGTACTTATTCAGACAGTGCCTCAAACTACTTCCCTTGGTATAAGCAAGAACTTGGAAAAGG
ACCTCAGCTTATTATAGACATTCGTTCAAATGTGGGCGAAAAGAAAGACCAACGAATTGCTG
TTACATTGAACAAGACAGCCAAACATTTCTCCCTGCACATCACAGAGACCCAACCTGAAGAC
TCGGCTGTCTACTTCTGTGCAGCAAGTACTTCGGGTGGTACTAGCTATGGAAAGCTGACATT
TGGACAAGGGACCATCTTGACTGTCCATCCAAATATCCAGAACCCTGACCCTGCCGTGTACC
AGCTGAGAGACTCTAAATCCAGTGACAAGTCTGTCTGCCTATTCACCGATTTTGATTCTCAA
ACAAATGTGTCACAAAGTAAGGATTCTGATGTGTATATCACAGACAAAACTGTGCTAGACAT
GAGGTCTATGGACTTCAAGAGCAACAGTGCTGTGGCCTGGAGCAACAAATCTGACTTTGCAT
GTGCAAACGCCTTCAACAACAGCATTATTCCAGAAGACACCTTCTTCCCCAGCCCAGAAAGT
TCCTGTGATGTCAAGCTGGTCGAGAAAAGCTTTGAAACAGATACGAACCTAAACTTTCAAAA
CCTGTCAGTGATTGGGTTCCGAATCCTCCTCCTGAAAGTGGCCGGGTTTAATCTGCTCATGAC
GCTGCGGCTGTGGTCCAGCTGAggatccctcgagagag

MASIRAVFIFLWLQLDLVNGENVEQHPSTLSVQEGDSAVIKCTYSDSASN 50
YFPWYKQELGKGPQLIIDIRSNVGEKKDQRIAVTLNKTAKHFSLHITETQ 100
PEDSAVYFCAASTSGGTSYGKLTFGQGTILTVHPNIQNPDPAVYQLRDSK 150
SSDKSVCLFTDFDSQTNVSQSKDSDVYITDKTVLDMRSMDFKSNSAVAWS 200

NKSDFACANAFNNSIIPEDTFFPSPESSCDVKLVEKSFETDTNLNFQNLS 250
VIGFRILLLKVAGFNLLMTLRLWSS*GSLER

TCR beta chain
ATGGGCCCCCAGCTCCTTGGCTATGTGGTCCTTTGCCTTCTAGGAGCAGGCCCCCTGGAAGCCCA
AGTGACCCAGAACCCAAGATACCTCATCACAGTGACTGGAAAGAAGTTAACAGTGACTTGT
TCTCAGAATATGAACCATGAGTATATGTCCTGGTATCGACAAGACCCAGGGCTGGGCTTAAG
GCAGATCTACTATTCAATGAATGTTGAGGTGACTGATAAGGGAGATGTTCCTGAAGGGTACA
AAGTCTCTCGAAAAGAGAAGAGGAATTTCCCCCTGATCCTGGAGTCGCCCAGCCCCAACCA
GACCTCTCTGTACTTCTGTGCCAGCAGTTTGGGGAGCTCCTACGAGCAGTACTTCGGGCCGG
GCACCAGGCTCACGGTCACAGAGGACCTGAAAAACGTGTTCCCACCCGAGGTCGCTGTGTTT
GAGCCATCAGAAGCAGAGATCTCCCACACCCAAAAGGCCACACTGGTGTGCCTGGCCACAG
GCTTCTACCCCGACCACGTGGAGCTGAGCTGGTGGGTGAATGGGAAGGAGGTGCACAGTGG
GGTCAGCACAGACCCACAGCCCCTCAAGGAGCAGCCCGCCCTCAATGACTCCAGATACTGC
CTGAGCAGCCGCCTGAGGGTCTCGGCCACCTTCTGGCAGAACCCCCGCAACCACTTCCGCTG
TCAAGTCCAGTTCTACGGGCTCTCGGAGAATGACGAGTGGACCCAGGATAGGGCCAAACCT
GTCACCCAGATCGTCAGCGCCGAGGCCTGGGGTAGAGCAGACTGTGGCTTCACCTCCGAGTC
TTACCAGCAAGGGGTCCTGTCTGCCACCATCCTCTATGAGATCTTGCTAGGGAAGGCCACCT
TGTATGCCGTGCTGGTCAGTGCCCTCGTGCTGATGGCCATGGTCAAGAGAAAGGATTCCAGAGG
CTAGcgatccctcgagagag

MGPQLLGYVVLCLLGAGPLEAQVTQNPRYLITVTGKKLTVTCSQNMNHEY 50
MSWYRQDPGLGLRQIYYSMNVEVTDKGDVPEGYKVSRKEKRNFPLILESP 100
SPNQTSLYFCASSLGSSYEQYFGPGTRLTVTEDLKNVFPPEVAVFEPSEA 150
EISHTQKATLVCLATGFYPDHVELSWWVNGKEVHSGVSTDPQPLKEQPAL 200
NDSRYCLSSRLRVSATFWQNPRNHFRCQVQFYGLSENDEWTQDRAKPVTQ 250
IVSAEAWGRADCGFTSESYQQGVLSATILYEILLGKATLYAVLVSALVLM 300
AMVKRKDSRG*RSLER

## Claims

1. A composition comprising an effector T cell transiently transfected with RNA encoding a T cell receptor (TCR) specific for an antigen, wherein the T cell demonstrates effector function specific for cells presenting the antigen in complex with an MHC molecule.

2. The composition of claim 1, wherein the antigen is a tumor antigen, a pathogen antigen or a self-antigen.

3. The composition of claim 2, wherein
(i) the antigen is a tumor antigen, preferably the tumor is renal cell carcinoma, melanoma, chronic lymphocytic leukemia, breast cancer, lung cancer, prostate cancer, ovarian cancer or colon cancer, and/or the antigen is MART-1, MAGE-1, MAGE-3 gp75, MDM2, tyrosinase, telomerase, gp100, survivin, alpha-1 fetoprotein, G250, or NY-ESO-1;
(ii) the antigen is a pathogen antigen, preferably the pathogen is HIV or HCV.

4. The composition of claim 1, wherein
(i) the effector function is one or more functions selected from the group consisting of IL-2 secretion, TNFα secretion, TNFβ secretion, interferon-γ (IFNγ) secretion, cytotoxicity, and regulatory effector function; or
(ii) the T cell is CD8+, preferably the TCR is MHC class I restricted, or the TCR is MHC class II restricted, or the effector function is cytotoxicity; or
(iii) the T cell is CD4+, preferably the TCR is MHC class I restricted, or the TCR is MHC class II restricted, or the effector function is activation of macrophages and/or activation of B cells, or the T cell is transiently transfected with RNA encoding FoxP3; or
(iv) said T cell is cyropreserved post electroporation.

5. The composition of claim 4, wherein the CD4+ T cell is
(i) a regulatory T cell (T_{reg}), preferably the effector function is regulatory effector function, most preferably the regulatory function is IL-10 secretion and/or TGF-β secretion, or the T_{reg} is CD4+CD25+, or the T cell is FoxP3+, or said TCR is specific for a self antigen; or
(ii) a T_{H}1 cell;
(iii) a T_{H}2 cell.

6. Use of the composition of any one of claims 1 to 5 for the production of a medicament for immunotherapy.

7. A method for imparting a new antigen specificity to a T cell, comprising electroporating a composition comprising purified CD8+ or purified CD4+ T cells with RNA encoding a TCR receptor specific for an antigen.

8. The method of claim 7, wherein
(i) the T cells are purified by magnetic sorting; or
(ii) the purified T cells comprise at least 75% of all T cells present in the composition, preferably at least 90% of all T cells present in the composition; or
(iii) the purified T cells have not been stimulated *in vitro* by phytohemagluttinin (PHA) or OKT3 prior to electroporation; or
(iv) the purified T cells are stimulated to proliferate prior to electroporation, preferably the purified T cells are stimulated with PHA and/or OKT3; or
(v) the T cells are purified by separation from T regulatory cells; or
(vi) the T cells are electroporated at a field strength of 100V/mm-150V/mm for 2-10 ms using a square wave pulse.

9. A method for imparting a new antigen specificity to T cells, comprising: electroporating resting T cells with RNA encoding a TCR specific for an antigen.

10. The method of claim 9, wherein
(i) the resting T cells are either purified CD8+ T cell or purified CD4+ T cells; or
(ii) the cells are purified by magnetic cell sorting; or
(iii) the T cells are electroporated at a field strength of 100V/mm-150V/mm for 2-10 ms using a square wave pulse.

11. A method for transiently transfecting T cells, comprising electroporating T cells with RNA a field strength of 100V/mm-150V/mm for 2-10 ms using a square wave pulse, wherein the T cells have not been stimulated in vitro by PHA or OKT3 prior to electroporation.

12. The method of claim 11, wherein
(i) said T cells are purified CD8+ T cells; or
(ii) the T cells are purified CD4+ T cells, preferably the T cells are purified regulatory T cells; or
(iii) the RNA encodes FoxP3.

13. A T cell produced by the method of any one of claims 9 to 12.

14. Use of the T cell of claim 13 for the production of a medicament for immunotherapy.

15. A method of providing antigen-specific T cell effector function to a subject, comprising administering a T cell transiently transfected with RNA encoding a TCR specific for an antigen, wherein the T cell demonstrates effector function for cell presenting the antigen in complex with an MHC molecule.

16. The method of claim 15, wherein
(i) the effector function is cytotoxicity; or
(ii) the antigen is tumor-specific, preferably the administration is by intratumoral injection; or
(iii) the antigen is pathogen-specific; or
(iv) the T cell is autologous to the subject.

17. A method for making a T_{reg} cell, comprising transfecting a CD4+ T cell with a nucleic acid encoding FoxP3.

18. The method of claim 17, wherein the nucleic acid is an mRNA.

19. A T_{reg} cell comprising an exogenous RNA encoding FoxP3 and/or being produced by the method of claim 17 or 18.

20. Use of the T_{reg} cell of claim 19 for the production of a medicament for immunotherapy.
